# EUROPEAN PATENT SPECIFICATION

(11) **EP 2 589 381 B1**
(45) Date of publication and mention of the grant of the patent: **31.08.2016**
(21) Application number: 11250938.5
(22) Date of filing: 23.12.2011
(51) Int. Cl.: A61K 31/198, A61P 11/00, A61P 11/06, A61K 9/46, A61K 45/06

(54) **Compositions for improving or preserving lung function in a patient with a pulmonary disorder**
Zusammensetzungen zur Verbesserung oder Aufrechterhaltung der Lungenfunktion bei einem Patienten mit einer Lungenerkrankung
Compositions pour améliorer ou préserver une fonction de poumons chez un patient souffrant de troubles pulmonaires

(30) Priority: 04.11.2011 US 201161555678 P
(43) Date of publication of application: 08.05.2013
(73) Proprietor: Tirouvanziam, Rabindra, Decatur GA 30030 (US); Conrad, Carol, Menlo Park CA 94025 (US); Herzenberg, Leonore A., Stanford CA 94305 (US)
(72) Inventor: Tirouvanziam, Rabindra, Decatur GA 30030 (US); Conrad, Carol, Menlo Park CA 94025 (US); Herzenberg, Leonore A., Stanford CA 94305 (US)
(74) Representative: Beresford, Keith Denis Lewis

(56) References cited:
- US-A1- 2007 049 641
- US-A1- 2008 107 746
- US-A1- 2009 192 227
- TIROUVANZIAM RABINDRA ET AL: "High-dose oral N-acetylcysteine, a glutathione prodrug, modulates inflammation in cystic fibrosis.", PROCEEDINGS OF THE NATIONAL ACADEMY OF SCIENCES OF THE UNITED STATES OF AMERICA 21 MAR 2006 LNKD- PUBMED:16537378, vol. 103, no. 12, 21 March 2006 (2006-03-21), pages 4628-4633, XP002677771, ISSN: 0027-8424
- DECRAMER MARC ET AL: "Effects of N-acetylcysteine on outcomes in chronic obstructive pulmonary disease (Bronchitis Randomized on NAC Cost-Utility Study, BRONCUS): a randomised placebo-controlled trial.", LANCET 2005 APR 30-MAY 6 LNKD- PUBMED:15866309, vol. 365, no. 9470, 30 April 2005 (2005-04-30), pages 1552-1560, XP27764348, ISSN: 1474-547X
- DECRAMER M ET AL: "Mucoactive therapy in COPD.", EUROPEAN RESPIRATORY REVIEW : AN OFFICIAL JOURNAL OF THE EUROPEAN RESPIRATORY SOCIETY JUN 2010 LNKD- PUBMED:20956182, vol. 19, no. 116, June 2010 (2010-06), pages 134-140, XP002677773, ISSN: 1600-0617
- SADOWSKA ET AL: "Antioxidant and anti-inflammatory efficacy of NAC in the treatment of COPD: Discordant in vitro and in vivo dose-effects: A review", PULMORNARY PHARMACOLOGY & THERAPEUTICS, ACADEMIC PRESS, GB, vol. 20, no. 1, 1 November 2006 (2006-11-01), pages 9-22, XP005731988, ISSN: 1094-5539, DOI: 10.1016/J.PUPT.2005.12.007
- DEKHUIJZEN P N R ET AL: "The role for N-acetylcysteine in the management of COPD.", INTERNATIONAL JOURNAL OF CHRONIC OBSTRUCTIVE PULMONARY DISEASE 2006 LNKD- PUBMED:18046886, vol. 1, no. 2, 2006, pages 99-106, XP9160301, ISSN: 1176-9106
- SUTHERLAND E RAND ET AL: "N-acetylcysteine and exacerbations of chronic obstructive pulmonary disease.", COPD DEC 2006 LNKD- PUBMED:17361500, vol. 3, no. 4, December 2006 (2006-12), pages 195-202, XP9160253, ISSN: 1541-2555
- DEMEDTS MAURITS ET AL: "High-dose acetylcysteine in idiopathic pulmonary fibrosis.", THE NEW ENGLAND JOURNAL OF MEDICINE 24 NOV 2005 LNKD- PUBMED:16306520, vol. 353, no. 21, 24 November 2005 (2005-11-24), pages 2229-2242, XP002677776, ISSN: 1533-4406
- MILLMAN M ET AL: "USE OF ACETYLCYSTEINE IN BRONCHIAL ASTHMA - ANOTHER LOOK", ANNALS OF ALLERGY, MCLEAN, VA, US, vol. 54, no. 4, 1 April 1985 (1985-04-01), pages 294-296, XP000992551, ISSN: 0003-4738
- DATABASE WPI Week 200822 Thomson Scientific, London, GB; AN 2008-D03147 XP002677777, & CN 101 049 293 A (HUANG Z) 10 October 2007 (2007-10-10)
- DATABASE WPI Week 200823 Thomson Scientific, London, GB; AN 2008-D16837 XP002677778, & CN 101 053 566 A (HUANG Z) 17 October 2007 (2007-10-17)

## Description

### CROSS REFERENCE TO RELATED APPLICATIONS

This application claims the benefit of priority to U.S. Application No. 12/420,577 (filed April 8, 2009), entitled "N-Acetylcysteine Compositions And Methods For Treating Acute Exacerbations Of Inflammatory Lung Disease," which is a continuation-in part of U.S. Application No. 11/507,706 (filed August 22, 2006), which claims the benefit of priority to U.S. Provisional Application No. 60/710,807 (filed Aug. 24, 2005) entitled "Methods For Treating And Monitoring Inflammation And Redox Imbalance In Cystic Fibrosis."

### FIELD OF THE INVENTION

The present invention relates to N-acetylcysteine compositions for use in methods of treatment for improving or preserving lung function in lung disease.

### BACKGROUND

### Oxidative Stress Associated With GSH Depletion

A free radical is a highly reactive and usually short-lived molecular fragment with one or more unpaired electrons. Free radicals are highly chemically reactive molecules. Because a free radical needs to extract a second electron from a neighboring molecule to pair its single electron, it often reacts with other molecules, which initiates the formation of many more free radical species in a self-propagating chain reaction. This ability to be self-propagating makes free radicals highly toxic to living organisms.

Living systems under normal conditions produce the vast majority of free radicals and free radical intermediates. They handle free radicals formed by the breakdown of compounds through the process of metabolism. Most reactive oxygen species come from endogenous sources as by-products of normal and essential metabolic reactions, such as energy generation from mitochondria or detoxification reactions involving the cytochrome P-450 enzyme system. The major sources of free radicals, such as O₂⁻ and HNO₂⁻, are modest leakages from the electron transport chains of mitochondria, chloroplasts, and endoplasmic reticulum.

Reactive oxygen species ("ROS"), such as free radicals and peroxides, represent a class of molecules that are derived from the metabolism of oxygen and exist inherently in all aerobic organisms. The term "oxygen radicals" as used herein refers to any oxygen species that carries an unpaired electron (except free oxygen). The transfer of electrons to oxygen also may lead to the production of toxic free radical species. The best documented of these is the superoxide radical. Oxygen radicals, such as the hydroxyl radical (OH-) and the superoxide ion (O₂⁻) are very powerful oxidizing agents that cause structural damage to proteins, lipids and nucleic acids. The free radical superoxide anion, a product of normal cellular metabolism, is produced mainly in mitochondria because of incomplete reduction of oxygen. The superoxide radical, although unreactive compared with many other radicals, may be converted by biological systems into other more reactive species, such as peroxyl (ROO⁻), alkoxyl (RO⁻) and hydroxyl (OH⁻) radicals.

The major cellular sources of free radicals under normal physiological conditions are the mitochondria and inflammatory cells, such as granulocytes, macrophages, and some T-lymphocytes, which produce active species of oxygen via the nicotinamide adenine nucleotide oxidase (NADPH oxidase) system, as part of the body's defense against bacterial, fungal or viral infections.

Oxidative injury may lead to widespread biochemical damage within the cell. The molecular mechanisms responsible for this damage are complex. For example, free radicals may damage intracellular macromolecules, such as nucleic acids (e.g., DNA and RNA), proteins, and lipids. Free radical damage to cellular proteins may lead to loss of enzymatic function and cell death. Free radical damage to DNA may cause problems in replication or transcription, leading to cell death or uncontrolled cell growth. Free radical damage to cell membrane lipids may cause the damaged membranes to lose their ability to transport oxygen, nutrients or water to cells.

Biological systems protect themselves against the damaging effects of activated species by several means. These include free radical scavengers and chain reaction terminators; "solid-state" defenses, and enzymes, such as superoxide dismutase, catalase, and the glutathione peroxidase system.

Free radical scavengers/chemical antioxidants, such as vitamin C and vitamin E, counteract and minimize free radical damage by donating or providing unpaired electrons to a free radical and converting it to a nonradical form. Such reducing compounds may terminate radical chain reactions and reduce hydroperoxides and epoxides to less reactive derivatives.

The term "solid state defense" as used herein refers to the mechanism whereby a macromolecule binds a radical-generating compound, de-excites an excited state species, or quenches a free radical. The most important solid-state defense in the body is the black pigment melanin, which scavenges odd electrons to form stable radical species, thus terminating radical chain reactions.

Enzymatic defenses against active free radical species include superoxide dismutase, catalases, and the glutathione reductase/peroxidase system. Superoxide dismutase (SOD) is an enzyme that destroys superoxide radicals. Catalase, a heme-based enzyme that catalyses the breakdown of hydrogen peroxide into oxygen and water, is found in all living cells, especially in the peroxisomes, which, in animal cells, are involved in the oxidation of fatty acids and the synthesis of cholesterol and bile acids. Hydrogen peroxide is a byproduct of fatty acid oxidation and is produced by white blood cells to kill bacteria.

Glutathione, a tripeptide composed of glycine, glutamic acid, and cysteine that contains a nucleophilic thiol (SH) group, is widely distributed in animal and plant tissues. It exists in both the reduced thiol form (GSH) and the oxidized disulfide form (GSSG). In its reduced GSH form, glutathione acts as a substrate for the enzymes GSH-S-transferases and GSH peroxidases, both of which catalyze reactions for the detoxification of xenobiotic compounds, and for the reduction of reactive oxygen species and other free radicals. The term "xenobiotic" is used herein to refer to a chemical which is not a natural component of the organism exposed to it.

Examples of xenobiotics include, but are not limited to, carcinogens, toxins and drugs. The metabolism of xenobiotics usually involves two distinct stages. Phase I metabolism involves an initial oxidation, reduction or dealkylation of the xenobiotic by microsomal cytochrome P-450 monooxygenases (Guengerich, F.P. Chem. Res. Toxicol. 4: 391-407 (1991); this step is often needed to provide hydroxyl- or amino groups, which are essential for phase II reactions. Glutathione detoxifies many highly reactive intermediates produced by cytochrome P450 enzymes in phase I metabolism. Without adequate GSH, the reactive toxic metabolites produced by cytochrome P-450 enzymes may accumulate causing organ damage.

Phase II metabolism generally adds hydrophilic moieties, thereby making a toxin more water soluble and less biologically active. Frequently involved phase II conjugation reactions are catalyzed by glutathione S-transferases (Beckett, G.J. & Hayes, J.D., Adv. Clin. Chem. 30: 281-380 (1993), sulfotransferases (Falany, CN, Trends Pharmacol. Sci. 12: 255-59 (1991), and UDP-glucuronyl-transferases (Bock, KW, Crit. Rev. Biochem. Mol. Biol. 26: 129-50 (1991). Glutathione S-transferases catalyze the addition of aliphatic, aromatic, or heterocyclic radicals as well as epoxides and arene oxides to glutathione. These glutathione conjugates then are cleaved to cysteine derivatives primarily by renal enzymes and then acetylated, thus forming N-acetylcysteine derivatives. Examples of compounds transformed to reactive intermediates and then bound to GSH include, but are not limited to, bromobenzene, chloroform, and acetaminophen. Such toxicants may deplete GSH.

Depletion of GSH may diminish the body's ability to defend against lipid peroxidation. Glutathione is a cofactor for Glutathione peroxidase (GPx), an enzyme of the oxidoreductase class, which catalyzes the detoxifying reduction of hydrogen peroxide and organic peroxides via oxidation of glutathione. GSH is oxidized to the disulfide linked dimer (GSSG), which is actively pumped out of cells and becomes largely unavailable for reconversion to reduced glutathione. Loss of large amounts of GSH results in cell death, while loss of smaller amounts can change cell function.

Thus, unless glutathione is resynthesized through other pathways, utilization of oxidized glutathione is associated with a decrease in the amount of glutathione available.

Glutathione reductase, a flavoprotein enzyme of the oxidoreductase class, is essential for the maintenance of cellular glutathione in its reduced form (Carlberg & Mannervick, J. Biol. Chem. 250: 5475-80 (1975)). It catalyzes the reduction of oxidized glutathione (GSSG) to reduced glutathione (GSH) in the presence of NADPH and maintains a high intracellular GSH/GSSG ratio of about 500 in red blood cells.

Synthesis of GSH requires cysteine, a conditionally essential amino acid that must be obtained from dietary sources or by conversion of dietary methionine via the cystathionase pathway. If the supply of cysteine is adequate, normal GSH levels are maintained. But GSH depletion occurs if supplies of cysteine are inadequate to maintain GSH homeostasis in the face of increased GSH consumption. Acute GSH depletion causes severe -- sometimes fataloxidative and/or alkylation injury, and chronic or slow arising GSH deficiency due to administration of GSH-depleting drugs, such as acetaminophen, or to diseases and conditions that deplete GSH, may be similarly debilitating.

Cysteine is necessary to replenish GSH. Although various forms of cysteine and its precursors have been used as nutritional and therapeutic sources of cysteine, N-acetylcysteine (NAC) is the most widely used and extensively studied. NAC is about 10 times more stable than cysteine and much more soluble than the stable cysteine disulfide, cystine. Glutathione, glutathione monoethyl ester, and L-2-oxothiazolidine-4-carboxylate (procysteine/OTC) also have been used effectively in some studies. In addition, dietary methionine and S-adenosylmethionine are an effective source of cysteine.

It is well known that NAC, as a cysteine prodrug, promotes cellular glutathione production, and thus decreases, or even prevents, oxidant-mediated damage. In addition, NAC may act as a direct scavenger for oxidants. Treatment with NAC provides beneficial effects in a number of respiratory, cardiovascular, endocrine, infectious, and other disease settings as described in WO05/017094. For example, rapid administration of NAC is the standard of care for preventing hepatic injury in acetaminophen overdose. NAC administered intravenously in dogs has been shown to protect against pulmonary oxygen toxicity and against ischemic and reperfusion damage [Gillissen, A., and Nowak, A., Respir. Med. 92: 609-23, 613 (1998)]. NAC treatment also has been shown to decrease NF-κB activation, which in turn decreases neutrophilic inflammation in the lung.

### Antioxidant Therapy In Chronic Pulmonary Diseases

The lung exists in a high-oxygen environment, and together with its large surface area and blood supply, is highly susceptible to injury mediated by oxidative stress. Since reactive oxygen species (ROS) are constantly formed in the lung, and since oxygen metabolites are believed to play a predominant role in the pathogenesis of various pulmonary inflammatory disorders, antioxidant therapy would seem to be a rational approach to take in pulmonary diseases. Patients with acute respiratory distress syndrome (ARDS), idiopathic pulmonary fibrosis (IPF), or chronic obstructive pulmonary disorder (COPD) have been the primary targets for clinical studies evaluating the efficacy of NAC in antioxidant therapy. The results have been, for the most part, inconclusive.

COPD, a syndrome of chronic airway inflammation, initiated in most cases by chronic tobacco smoke exposure, which damages the airways and lung parenchyma over many years, has been extensively studied in this regard. An accelerated functional deterioration is accompanied by the development of cough, sputum production, dyspnea, and abnormal gas exchange, and leads to an increasing risk of acute flares of disease referred to as exacerbations. Exacerbation frequency increases as the disease progresses, further accelerating lung function decline.

The presence of oxidative stress in the airways of smokers and patients with COPD has been shown by increased products of lipid peroxidation and altered antioxidant status. Patients with COPD are known to have increased numbers of activated neutrophils in their airways that are believed to be attracted to the airways by the cytokines IL-8 and TNF-α, which are present in increased levels in the lungs of patients with stable COPD. Drost, E.M., Skwarski, K.N., Sauleda, J., Soler, N., Roca, J., Agusti, A., MacNee, W. "Oxidative Stress and Airway Inflammation in Severe Exacerbations of COPD," Thorax 60: 293-300 (2005) disclose that exacerbations of COPD are considered to reflect worsening of the underlying chronic inflammation in the airways. They reported that increased oxidative stress in the airways of patients with COPD is increased further in severe and very severe exacerbations of the disease and is associated with increased neutrophil influx and levels of IL-8, an inflammatory cytokine associated with airway inflammation in COPD. The study acknowledged that in COPD, the interpretation of differences between exacerbations and the stable state may actually be a reflection of differences in disease severity, because exacerbations were studied in patients with severe and very severe underlying COPD and compared with stable patients with moderate disease.

While there is some evidence that oral NAC offsets chronic redox stress when administered in the long term for chronic respiratory conditions, some studies have demonstrated a beneficial effect, but others have not. For example, NAC has been used for over 20 years to treat COPD, a disease not characterized by glutathione deficiency. Gillissen and Nowak, Respir. Med. 92: 609-23, 615 (1998), for example, reported that improvements in glutathione levels were seen in patients with ARDS and IPF, but not COPD, who received 600-1800 mg NAC given daily by mouth. Oral NAC at high doses (generally 1.2 to 1.8 g/day) has been proposed for the treatment (preventive or symptomatic) of exacerbations in a subset of patients with COPD who are not receiving inhaled corticosteroids (Sutherland, E.R., et al., COPD Chronic Obstructive Pulmonary Disease 3: 195-202 (2006)). Although treatment with 600 mg oral NAC per day was ineffective at preventing deterioration in lung function and exacerbations in patients with COPD who had frequent exacerbations (i.e., at least two per year for 2 years), these investigators suggested that higher doses of NAC, such as 1200 mg or 1800 mg per day, could be assessed in future trials (Decramer, M., Lancet 365: 1552-60 (2005)). Oral NAC at high doses (generally 1.2 to 1.8 g/day) also has been proposed for the treatment (preventive or symptomatic) of exacerbations in chronic bronchitis, an inflammation, or irritation, of the airways in the lungs characterized by a chronic cough and chronic mucus production without another known cause (see Grandjean, E.M. et al., Clinical Therapeutics 22(2): 209-21 (2000), and Stey, C., et al., Eur. Resp. J. 16: 253-62 (2000)).

### Cystic Fibrosis

Cystic fibrosis (CF) is an inherited autosomal recessive disorder. It is one of the most common fatal genetic disorders in the United States, affecting about 30,000 individuals, and is most prevalent in the Caucasian population, occurring in one of every 3,300 live births. The gene involved in cystic fibrosis, which was identified in 1989, codes for a protein called the cystic fibrosis transmembrane conductance regulator (CFTR). CFTR is normally expressed by exocrine epithelia throughout the body and regulates the movement of chloride ions, bicarbonate ions and glutathione into and out of cells. In cystic fibrosis patients, mutations in the CFTR gene lead to alterations or total loss of CFTR protein function, resulting in defects in osmolarity, pH and redox properties of exocrine secretions. In the lungs, CF manifests itself by the presence of a thick mucus secretion which clogs the airways. In other exocrine organs, such as the sweat glands, CF may not manifest itself by an obstructive phenotype, but rather by abnormal salt composition of the secretions (hence the clinical sweat osmolarity test to detect CF patients).

The predominant cause of illness and death in cystic fibrosis patients is progressive lung disease. The thickness of CF mucus, which blocks the airway passages, is believed to stem from abnormalities in osmolarity of secretions, as well as from the presence of massive amounts of DNA, actin, proteases and prooxidative enzymes originating from a subset of inflammatory cells, called neutrophils. Indeed, CF lung disease is characterized by early, hyperactive neutrophil-mediated inflammatory reactions to both viral and bacterial pathogens.

The hyperinflammatory syndrome of CF lungs has several underpinnings, among which an imbalance between pro-inflammatory chemokines, chiefly IL-8, and anti-inflammatory cytokines, chiefly IL-10, seems to play a major role. See Chmiel et al. Clin Rev Allergy Immunol. 3(1):5-27 (2002). Chronic oxidative stress in CF patients may severely affect the deformability of blood neutrophils circulating in CF lung capillaries, thereby increasing their recruitment to the lungs. See Hogg. Physiol Rev. 67(4):1249-95 (1987). Chronic oxidative stress in CF is linked to the overwhelming release of oxidants by inflammatory lung neutrophils and to abnormal antioxidant defenses caused by malabsorption of dietary antioxidants through the gut and a possible defect in GSH efflux. See Wood et al. J. Am. Coll. Nutr. 20(2 Suppl):157-165 (2001).

The hyperinflammatory syndrome at play in CF lungs may predispose such patients to chronic infections with opportunistic bacterial pathogens. The most common bacterium to infect the CF lung is *Pseudomonas aeruginosa*, a gram-negative microorganism. The lungs of most children with CF become colonized by *P. aeruginosa* before their third birthday. By their tenth birthday, *P. aeruginosa* becomes dominant over other opportunistic pathogens. See Gibson et al., Am. J. Respir. Crit Care Med., 168(8): 918-951 (2003). *P. aeruginosa* infections further exacerbate neutrophilic inflammation, which causes repeated episodes of intense breathing problems in CF patients. Although antibiotics may decrease the frequency and duration of these attacks, the bacterium progressively establishes a permanent residence in CF lungs by switching to a so-called "mucoid" biofilm form of high resistance and low virulence, which never may be eliminated completely from the lungs. The continuous presence in CF lungs of inflammatory by-products, such as extracellular DNA and elastase, could play a major role in selecting for mucoid *P*. *aeruginosa* forms. See Walker et al. Infect Immun. 73(6): 3693-3701 (2005).

Treatments for CF lung disease typically involve antibiotics, anti-inflammatory drugs, bronchodilators, and chest physiotherapy to help fight infection, neutrophilic inflammation and obstruction and clear the airways. Nevertheless, the persistent, viscous and toxic nature of airway secretions in cystic fibrosis lung disease still leads to progressive deterioration of lung function. See Rancourt et al., Am. J. Physiol. Lung Cell Mol. Physiol. 286(5): L931-38 (2004).

Although it is characterized by heavy inflammation, CF historically was thought to be a mucus disease. N-acetylcysteine (NAC) is a widely used mucolytic drug in patients with a variety of disorders, including cystic fibrosis. See Rochat, et al., J. Cell Physiol. 201(1): 106-16 (2004). It has been hypothesized that NAC works as a mucolytic by rupturing the disulfide bridges of the high molecular weight glycoproteins present in the mucus, resulting in smaller subunits of the glycoproteins and reduced mucous viscosity. Id. To this end, researchers and clinicians have administered NAC to CF patients generally by nebulization, as well as orally. Two placebo-controlled studies have reported beneficial effects of oral NAC treatment on lung function in cystic fibrosis. See G. Stafanger, et al., Eur. Respir. J. 1(2): 161-67 (1988). Active treatment consisted of NAC administered as a 200 mg oral dose three times daily (for patients weighing less than 30 kg) or as a 400 mg oral dose two times daily (for patients weighing more than 30 kg). Ratjen, F., et al., Eur. J. Pediatr. 144(4): 374-78 (1985) reported improvement in some measures of lung function but saw no significant clinical differences between patients treated with oral NAC (200 mg 3 times a day), the secretolytic drug ambroxol (30 mg, three times daily), and placebo. A very short fourth study (2 weeks) failed to find any significant difference between the trial arms. See Gotz et al, Eur. J. Resp. Dis. 61 (Suppl) 111: 122-26 (1980).

Duijvestijn, Y.C. and Brand, P.L. Acta Paediatr. 88(1): 38-41 (1999) observed, however, that despite the fact that NAC commonly is used in CF, there is remarkably little published data on its effects. They tested their hypothesis that NAC's antioxidant properties could be useful in preventing decline of lung function (defined as forced expiratory volume in one second, or FEV1) in cystic fibrosis by performing a systematic review of the literature to evaluate whether published evidence supports the use of NAC administered orally or by nebulization to improve lung function in patients with cystic fibrosis. They identified 23 papers, the majority of which were uncontrolled clinical observations, of which only three randomized controlled trials on nebulized NAC were found. None of these studies showed a statistically significant or clinically relevant beneficial effect of NAC aerosol. They found a small beneficial effect of doubtful clinical relevance of oral NAC on FEV1 in CF. Although they suggested that the effects of long-term treatment with oral NAC on lung function in CF should be investigated, they concluded that there is no evidence supporting the use of N-acetylcysteine in cystic fibrosis.

Despite these findings, redox-based therapy is an attractive idea for CF, since redox imbalance is a well-recognized aspect of the disease, yet seldom considered as a therapeutic target. See Cantin, Curr Opin Pulm Med. 10(6):531-6 (2004). Systemic oxidative stress may affect blood neutrophils by lowering their intracellular GSH levels, which in turn renders them more prone to lung trapping and dysfunction. See Hogg. Physiol Rev. 67(4):1249-95 (1987). Besides, systemic oxidative stress may alter the chemokine/cytokine balance, favoring inflammation, which systemic NAC treatment may help alleviate. See Zafarullah et al. Cell Mol Life Sci. 60(1):6-20 (2003).

### Acute Exacerbations Of Pulmonary Disease

A systematic review of randomized controlled trials for established acute oxidative/inflammatory syndromes, such as Acute Respiratory Distress Syndrome (ARDS), which is characterized by diffuse inflammation of the lung's alveolar-capillary membrane in response to various pulmonary and extrapulmonary insults, and Acute Lung Injury (ALI), a milder form of lung injury, showed that NAC had no effect on early mortality in these diseases (Adhikari, N., Burns, KEA, Meade, MO, The Cochrane Library 1: 1-43, John Wiley & Sons, Ltd., 2008).

Acute exacerbations of CF are characterized by increased oxidative stress and sputum concentrations of bioactive lipid mediators. Reid, D. W., et al., Respirology 12(1): 63-69 (2007). McGrath, L.T. et al, "Oxidative stress during acute respiratory exacerbations in cystic fibrosis," Thorax 54: 518-523 (1999) have reported that during acute respiratory exacerbations, patients with CF are subject to acute oxidative attack in addition to the chronic systemic oxidative stress found in this condition. Such acute respiratory exacerbations in CF are characterized by increased respiratory symptoms, reduction in forced expiratory volume in one second ("FEV 1 ") of more than 10%, and a decision to treat with intravenous antibiotics. As reported, although almost all of the antioxidant scavengers developed to cope with the acute attack were partially depleted during infection, antibiotic treatment of the acute infection tended to reduce measures of free radical damage by moderating the infection and hence the immune response.

Like in CF, it is known that chronic phase and acute pathological flares of such chronic pulmonary diseases as Acute Respiratory Distress Syndrome (ARDS), Acute Lung Injury (ALI), Chronic Bronchitis (CB), and Chronic Obstructive Pulmonary Disease (COPD) share a common feature, i.e., their chronic phase and acute pathological flares are associated with redox and inflammatory dysfunctions and an increased proteolysis of lung tissue.

Unlike CF, ARDS, ALI, CB, and COPD, both Idiopathic Pulmonary Fibrosis (IPF) and Asthma are characterized by considerable matrix thickening/deposition in the mucosa/lumen of the airways. The effect of high-dose oral NAC has not been tested against acute exacerbations in either IPF or asthma.

Idiopathic Pulmonary Fibrosis (IPF), a syndrome regrouping several diseases with progressive fibrosis of the alveoli, is a chronic, progressive, incurable lung disease characterized by deposition of fibers in the lung through the hyperproliferation of myofibroblasts. Causative factors remain unknown. In some individuals, it develops quickly, while others have cryptic disease. An oxidant-antioxidant imbalance that depletes glutathione levels has been described in IPF.

A clinical study reported by Demedts, Maurits, et al., New England J. Med. 353 (21): 2229-42 (2005) has suggested that NAC may be beneficial when combined with standard therapies for chronic IPF, but the study was not powered to show the impact of NAC on survival, did not address use of NAC as a primary therapy in IPF patients, and did not address the effect of high-dose oral NAC on acute exacerbations of IPF. The double-blind, randomized, placebo-controlled multicenter study assessed the effectiveness over one year of 600 mg NAC administered three times daily added to standard therapy with prednisone plus azothioprine to test whether this regimen would slow the functional deterioration in patients with IPF has been reported. The primary endpoints were changes between baseline and month 12 in vital capacity (meaning the total amount of air that may be exhaled after a maximum inspiration) and in single-breath carbon monoxide diffusing capacity ("DL_{CO}"). The results of the study showed that NAC plus standard therapy (prednisone plus azothioprine) slows the deterioration of the primary endpoints vital capacity and DL_{CO} in patients with IPF better than does the standard therapy (prednisone plus azothioprine) alone.

Episodes of idiopathic acute respiratory deterioration have been termed acute exacerbations of IPF. Collard, H.R. et al., Am. J. Respir. Crit. Care med. 176(7): 636-43 (2007). The etiology of acute exacerbations of IPF is unknown. There are several competing hypotheses, including, but not limited to, the hypothesis that acute exacerbations of IPF represents a distinct, pathobiological manifestation of the primary disease process, characterized by idiopathic lung injury; the hypothesis that acute exacerbations of IPF may represent clinically occult but biologically distinct conditions that go undiagnosed, such as viral infection, or aspiration; and the hypothesis that acute exacerbations of IPF may be the sequelae of an acute direct stress to the lung, with a subsequent acceleration of the already abnormal fibroproliferative process intrinsic to IPF.

Asthma is an inflammatory disease of the lungs characterized by reversible (in most cases) airway obstruction due to narrowing of the conducting airways, hyper-responsiveness/hyper-reactivity, and chronic inflammation characterized by an influx and activation of inflammatory cells, generation of inflammatory mediators, and epithelial cell shedding. In chronic asthma, there is an increased sequestration within the lungs of leukocytes from the peripheral microcirculation. Since many chronic asthma patients have eosinophilic infiltrates, eosinophils are thought to play a critical role in the inflammatory response in chronic asthma. Indeed, it is believed that much of the lung problems in chronic asthma relates to the eosinophil disease. In addition, neutrophils isolated from peripheral blood of asthmatic patients generate greater amounts of reactive oxygen species than cells from normal subjects, may be involved in acute exacerbations of asthma. (Kirkham, P., Rahman, I., Pharmacology & Therapeutics 111: 476-94 (2006)).

Oxidative stress is believed to play a key role in the pathogenesis of clinically stable (chronic) bronchial asthma. It also has been shown that acute exacerbations of asthma [meaning a sudden increase in breathlessness over the preceding 48 hours and presence of one of the following signs: tachypnea (meaning a respiratory rate of > 18), use of accessory muscles or respiration, audible wheezing, prolonged expiration with rhonchi (meaning a sound occurring during inspiration or expiration caused by air passing through bronchi that are narrowed by inflammation, spasm of smooth muscle, or presence of mucus in the lumen heard on auscultation (meaning a diagnostic method of listening to the sounds made) of the chest] are associated with increased inflammation in the airways and with increased oxidative stress. Nadeem, A., et al., J. Asthma 1:45-50 (2005).

Asthmatic exacerbations commonly occur in two phases: an immediate phase, caused by release of mediators, that often is characterized by bronchoconstriction resulting in wheezing and coughing, and an inflammatory or late phase, that includes increasing airway inflammation, which leads to hyper-responsiveness.

There are many published guidelines for management of asthma available, but there is little if any documented objective data to support their usefulness in acute care of asthma.

Although chronic redox and inflammatory stresses in asthma (Nadeem, 2005; Kirkham 2006) have been documented, the effect of high-dose oral NAC has not been tested against acute exacerbations in asthma.

Tuberculosis (TB), once believed to have been almost eradicated, has shown a resurgence and a substantial increase in drug resistance. Human immunodeficiency virus (HIV) infection is a major risk factor for the development of TB, and TB seems to make HIV infection worse [Sacchetini, J.C., et al. Nat. Rev. Microbiol. 6(1):41-52 (2008)]. Immune reconstitution inflammatory syndrome (referred to herein as IRS or IRIS), is an adverse consequence of the restoration of pathogen-specific immune responses in HIV infected patients during the initial months of highly active anti-retroviral therapy. Symptoms include fever, lymphadenopathy, and worsening of respiratory and other TB symptoms. Although the pathophysiology of IRIS is unknown, preliminary investigations suggest that an acute exacerbation of mycobacterium-specific Th1 responses against mycobacterial antigens after HIV infection control by this therapy may cause IRIS in HIV/TB patients. See Bougarit, A. et al., AIDS 20: F1-F7 (2006); Shankar, E.M., AIDS Research & Therapy 4: 29 (2007).

United States Patent Application Publication No. US 2007/0049641 A1 discloses pharmaceutical kits and methods to treat lung inflammation and redox imbalance in human cystic fibrosis patients using pharmaceutical compositions containing N-acetylcysteine, pharmaceutically acceptable salts of N-acetylcysteine, or N-acetylcysteine derivatives.

United States Patent Application Publication No. US 2009/0192227 A1 discloses the use of N-acetylcysteine compositions and methods for treating inflammation and redox imbalance in acute exacerbations of inflammatory lung disease such as cystic fibrosis.

The present invention describes use of NAC as a primary therapy to improve or preserve lung function in patients suffering from a pulmonary disorder that includes an inflammatory component.

### SUMMARY OF THE INVENTION

The invention relates to a pharmaceutical composition for use in the improvement of lung function as claimed. The invention provides a pharmaceutical composition comprising (a) a therapeutically effective amount of N-acetylcysteine or a pharmaceutically acceptable salt of N-acetylcysteine and (b) a carrier for use in treating a patient suffering from cystic fibrosis (CF) that comprises (i) lung inflammation, measured by sputum elastase activity measured in sputum, sputum human neutrophil elastase (HNE) activity, and sputum IL-8 levels; and GSH in whole blood; (ii) decreasing lung function measured as FEV1, FVC and FEF, and (iii) incidence of pulmonary and sinus exacerbations, wherein the therapeutic amount is effective to increase Forced Expiratory Volume in one second (FEV₁) by at least 0.15% over a baseline Forced Expiratory Volume in one second (FEV₁) value and thereby improve or preserve a lung function in the patient measured as FEV1 (p=0,02) for 6-months, compared to a significant loss of function in placebo recipients.

According the disclosure the composition is capable of acute or chronic administration. According the disclosure the pharmaceutical composition is a tablet. According to the disclosure the pharmaceutical composition is an effervescent tablet. According to another disclosure each dose of the pharmaceutical composition is individually wrapped to avoid oxidation. According to another disclosure the pharmaceutical composition is formulated for oral administration. According to another disclosure the pharmaceutical composition is formulated for parenteral, intravenous, intratracheal, intramuscular, or intraperitoneal administration. According to another disclosure the therapeutically effective amount comprises at least 200 mg of N-acetylcysteine, or the pharmaceutically acceptable salt of N-acetylcysteine but less than 20,000 mg of N-acetylcysteine, or the pharmaceutically acceptable salt of N-acetylcysteine. According to another disclosure the therapeutically effective amount ranges from about 900 mg per day of N-acetylcysteine, or the pharmaceutically acceptable salt of N-acetylcysteine to about 2,700 mg per day of N-acetylcysteine, or the pharmaceutically acceptable salt of N-acetylcysteine. According to another embodiment, the therapeutically effective amount for oral administration is about 900 mg of N-acetylcysteine, or the pharmaceutically acceptable salt of N-acetylcysteine each time, three times a day. The method of treatment using the pharmaceutical composition of the invention further comprises monitoring at least one lung function before and at a plurality of time points during treatment. Preferably, monitoring at least one lung function is carried out at 4, 8, 12, 16, 20, and 24 weeks following treatment. According to another option improved lung function of the patient is maintained for at least 6 months following administration of the therapeutically effective amount of N-acetylcysteine, or the pharmaceutically acceptable salt of N-acetylcysteine. According to another option the pharmaceutical composition increases Forced Expiratory Volume in one second (FEV1) of the patient by at least 4% over the Forced Expiratory Volume in one second (FEV1) of the untreated control patient. According to another option the pharmaceutical composition increases Forced Expiratory Volume in one second (FEV1) of the patient by at least 2% over a baseline Forced Expiratory Volume in one second (FEV1) value of the patient measured before treatment. According to another option the pharmaceutical composition increases time between exacerbations in the patient compared to a control. According to another option the pharmaceutical composition further comprises an anti-infective agent, bronchodilating agent, or anti-inflammatory agent. According to another embodiment, the pharmaceutical composition increases Forced Expiratory Volume in one second (FEV₁) of a patient treated with the pharmaceutical composition by a proportion in the range from 4% to 6% over the FEV₁ value of an untreated control patient. According to another embodiment, the pharmaceutical composition increases Forced Expiratory Volume in one second (FEV₁) of a patient treated with the pharmaceutical composition by a proportion in the range from 0.15% to 2% over a baseline Forced Expiratory Volume in one second (FEV₁) value of the patient measured before treatment.

### BRIEF DESCRIPTION OF THE DRAWINGS

**FIGURE 1** shows a diagram of a multi-center phase IIB randomized placebo-controlled, double-blind study designed for assessing the effects of oral N-acetylcysteine in cystic fibrosis patients.
**FIGURE 2** shows baseline characteristics of cystic fibrosis patients enrolled in the phase IIB clinical study.
**FIGURE 3** shows baseline pulmonary function of cystic fibrosis patients enrolled in the phase IIB clinical study.
**FIGURE 4** shows primary and secondary outcomes of the oral administration of N-acetylcysteine in cystic fibrosis patients as compared to a placebo-treated group.
**FIGURE 5** shows a graph plotting the change in neutrophil elastase activity (ml/ml) measured at screening, week 12 and week 24 for NAC-treated (dark grey) and placebo-treated (light grey).
**FIGURE 6** shows a graph plotting the relative % change in functional expiratory volume in liters measured at screening, week 12 and week 24 for NAC-treated (dark grey) and placebo-treated (light grey).

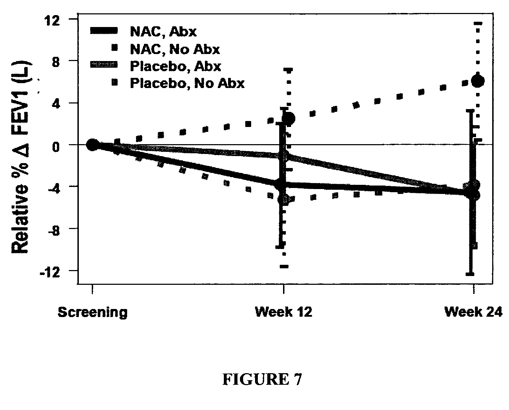

**FIGURE 7** shows a graph plotting the relative % change in functional expiratory volume in liters measured at screening, week 12 and week 24 for NAC+inhaled antibiotics-treated (solid dark grey), NAC-treated (dashed dark grey), placebo + inhaled antibiotics-treated (solid light grey) and placebo-treated (dashed light grey).

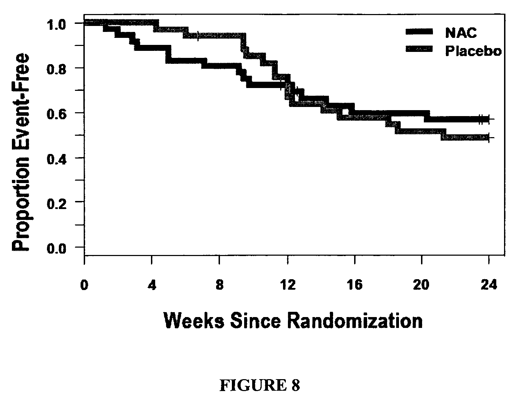

**FIGURE 8** shows a graph plotting the proportion of event-free pulmonary exacerbation over 24 weeks for NAC-treated (dark grey) and placebo-treated (light grey).

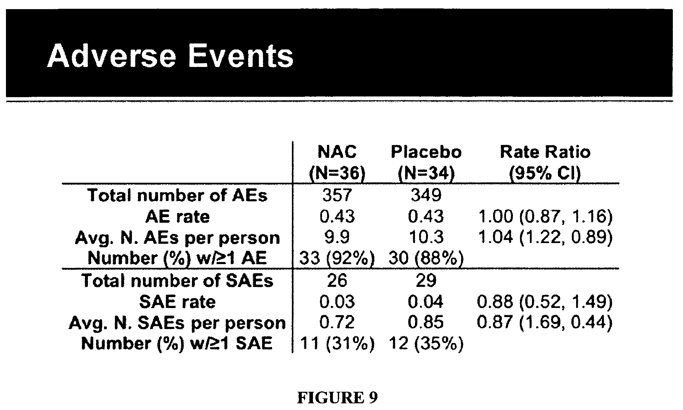

**FIGURE 9** shows adverse events measured in N-acetylcysteine-treated and placebo-treated groups.

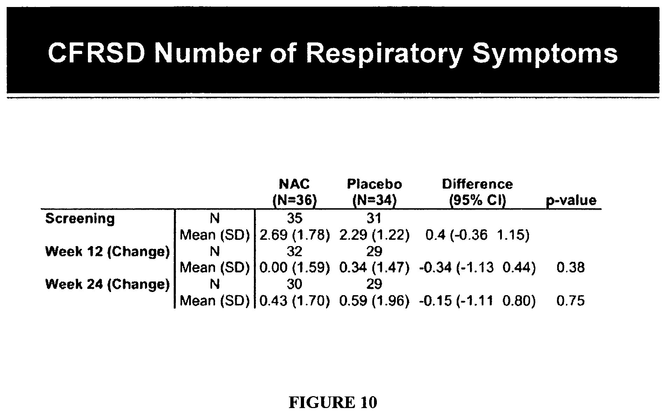

**FIGURE 10** shows cystic fibrosis respiratory symptoms diary ("CFRSD") results in N-acetylcysteine-treated and placebo-treated groups.

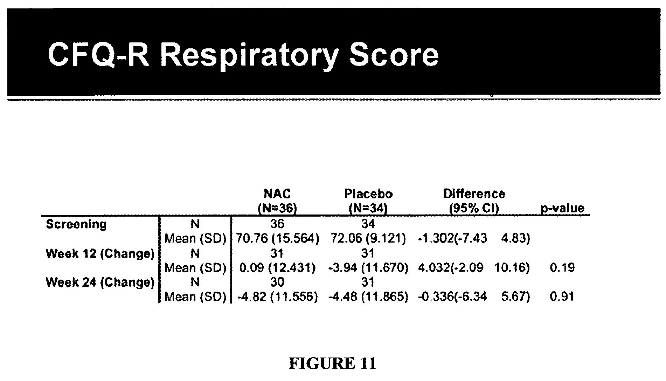

**FIGURE 11** shows cystic fibrosis questionnaire revised ("CFQ-R") respiratory score in N-acetylcysteine-treated and placebo-treated groups.

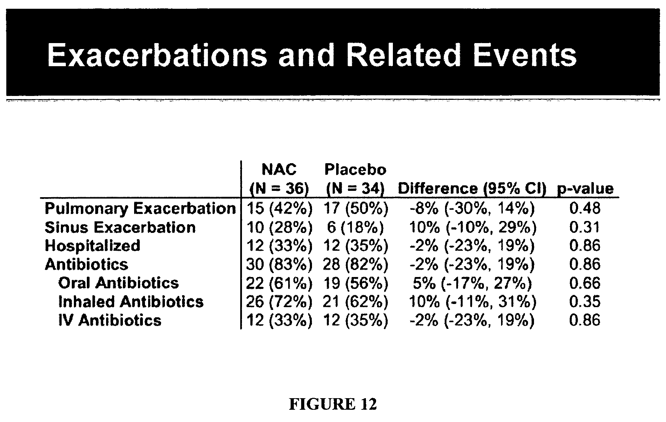

**FIGURE 12** shows exacerbations (pulmonary and sinus) and related events (hospitalized, antibiotic treatment -oral, inhaled and IV antibiotics) in N-acetylcysteine-treated and placebo-treated groups.

### DETAILED DESCRIPTION OF THE INVENTION

The present invention describes compositions and methods for improving or preserving lung function in a patient suffering from cystic fibrosis (CF).

The term "acute" as used herein refers to a rapid onset, brief (not prolonged), and severe health-related state, and to a plan of treatment designed to achieve certain results over a brief term.

The term "fibroblast growth factor (FGF)" refers to a family of over a dozen structurally related members. FGF1 is also known as acidic FGF; FGF2 is sometimes called basic FGF (bFGF); and FGF7 sometimes goes by the name keratinocyte growth factor. FGFs can activate a set of receptor tyrosine kinases called the fibroblast growth factor receptors (FGFRs). Receptor tyrosine kinases are proteins that extend through the cell membrane. When the FGF receptor binds an FGF (and only when it binds an FGF), the dormant kinase is activated, and phosphorylates certain proteins within the responding cell, activating those proteins. FGFs are associated with several developmental functions, including angiogenesis (blood vessel formation), mesoderm formation, and axon extension. While FGFs often can substitute for one another, their expression patterns give them separate functions. FGF2, for example, is especially important in angiogenesis.

The term "chronic" refers to a persistent, long-term, health-related state of 3 months duration or longer and to a plan of treatment designed to achieve certain results over a long term.

The term "condition," as used herein, refers to a variety of health states and is meant to include disorders or diseases, and inflammation caused by any underlying mechanism or disorder.

The term "disease" or "disorder," as used herein, refers to an impairment of health or a condition of abnormal functioning.

The terms "event" or "adverse event" are used interchangeably to refer to any undesirable experience associated with the use of a medical product in a patient.

The term "exacerbations" as used herein refers to an increase in the severity of a disease or any of its signs or symptoms. A pulmonary exacerbation, for example, is a worsening of respiratory signs and symptoms. The term "event free exacerbation" as used herein therefore refers to an exacerbation that is not connected to an adverse event.

The term "hypoxia-inducible factor (HIF-1) refers to an inducible transcriptional complex that plays an essential role in cellular responses to hypoxia. By hydroxylation of specific prolyl and asparinyl residues in the α subunit of HIF by a series of non-haem iron-dependent dioxygenases, transduces the oxygen-sensitive signal.

The term "idiopathic" refers to a disease of unknown cause.

The term interstitial lung disease ("ILD") includes a variety of chronic lung disorders in which lung tissue is damaged in some known or unknown way, the walls of the air sacs in the lung become inflamed; and scarring (or fibrosis) begins in the interstitium (or tissue between the air sacs) and the lung becomes stiff. When all known causes of interstitial lung disease have been ruled out, the condition is called idiopathic pulmonary fibrosis.

The term "inflammation" as used herein refers to the physiologic process by which vascularized tissues respond to injury. See, e.g., FUNDAMENTAL IMMUNOLOGY, 4th Ed., William E. Paul, ed. Lippincott-Raven Publishers, Philadelphia (1999) at 1051-1053. During the inflammatory process, cells involved in detoxification and repair are mobilized to the compromised site by inflammatory mediators. Inflammation is often characterized by a strong infiltration of leukocytes at the site of inflammation, particularly neutrophils (polymorphonuclear cells). These cells promote tissue damage by releasing toxic substances at the vascular wall or in uninjured tissue. Traditionally, inflammation has been divided into acute and chronic responses.

The term "acute inflammation" as used herein refers to the rapid, short-lived (minutes to days), relatively uniform response to acute injury characterized by accumulations of fluid, plasma proteins, and neutrophilic leukocytes. Examples of injurious agents that cause acute inflammation include, but are not limited to, pathogens (e.g., bacteria, viruses, parasites), foreign bodies from exogenous (e.g. asbestos) or endogenous (e.g., urate crystals, immune complexes), sources, and physical (e.g., burns) or chemical (e.g., caustics) agents.

The term "chronic inflammation" as used herein refers to inflammation that is of longer duration and which has a vague and indefinite termination. Chronic inflammation takes over when acute inflammation persists, either through incomplete clearance of the initial inflammatory agent or as a result of multiple acute events occurring in the same location. Chronic inflammation, which includes the influx of lymphocytes and macrophages and fibroblast growth, may result in tissue scarring at sites of prolonged or repeated inflammatory activity.

A "control," as used herein, can refer to an active, positive, negative or vehicle control. As will be understood by those of skill in the art, controls are used to establish the relevance of experimental results, and provide a comparison for the condition being tested.

The term "FEV1" or "Forced Expiratory Volume in One Second" as used herein refers to the volume of air, which can be forcibly exhaled from the lungs in the first second of a forced expiratory maneuver. It is expressed as liters. The term "FEV1/FVC" or "FEV1 Percent (FEV1%)" as used herein refers to the ratio of FEV1 to FVC. It indicates what percentage of the total FVC was expelled from the lungs during the first second of forced exhalation. This number is called FEV1%, %FEV1 or FEV1/FVC ratio. The term "FEV3" or "Forced Expiratory Volume in Three Seconds" as used herein refers to the volume of air which can be forcibly exhaled in three seconds, which is measured in Liters. This volume usually is fairly close to the FVC since, in the normal individual, most of the air in the lungs can be forcibly exhaled in three seconds. The term "FEV3/FVC" or "FEV3%" as used herein refers to the ratio of FEV3 to the FVC. It indicates what percentage of the total FVC was expelled during the first three seconds of forced exhalation. This is called "%FEV3" or "FEV3%".

The term "FEF" or "Forced Expiratory Flow" as used herein refers to a measure of how much air can be expired from the lungs. It is a flow rate measurement and is measured as liters/second or liters/minute. The FVC expiratory curve is divided into quartiles and therefore there is a FEF that exists for each quartile. The quartiles are expressed as FEF25%, FEF50%, and FEF75% of FVC.

The term "FVC" or "Forced Vital Capacity" as used herein refers to the volume of air, which can be forcibly and maximally exhaled out of the lungs until no more can be expired. FVC is usually expressed in units called liters.

The terms "lung function" or "pulmonary function" are used interchangeably herein refers to the functional status of the lungs. The primary instrument used in pulmonary function testing is the spirometer. It is designed to measure changes in volume and can only measure lung volume compartments that exchange gas with the atmosphere. Spirometers with electronic signal outputs (pneumotachs) also measure flow (volume per unit of time). A device is usually attached to the spirometer, which measures the movement of gas in and out of the chest and is referred to as a spirograph. Exemplary endpoints that reflect lung function include, but are not limited to, FEV1, FEF, FVC, etc.

The term "MVV" or "Maximal Voluntary Ventilation" as used herein refers to a value determined by having the patient breathe in and out as rapidly and fully as possible for 12 - 15 seconds. The total volume of air moved during the test can be expressed as L/sec or L/min. This test parameter reflects the status of the respiratory muscles, compliance of the thorax-lung complex, and airway resistance.

The term "PEFR" or "Peak Expiratory Flow Rate" as used herein refers to a maximum flow rate achieved by the patient during the forced vital capacity maneuver beginning after full inspiration and starting and ending with maximal expiration. It can either be measured in L/sec or L/min.

As used herein, the term "modulate" or "modulating" refers to adjusting, changing, or manipulating the function or status of at least one measure. Such modulation may be any change, including an undetectable change.

The term "oxidative stress" as used herein refers to a condition caused by an imbalance between reactive oxygen species and the antioxidant defense mechanisms of a cell, leading to an excess production of oxygen metabolites. Skaper, et al., Free Radical Biol. & Med. 22(4): 669-678 (1997).

The term "redox" as used herein refers to reduction and oxidation. Reduction may occur , for example, by (a) acceptance of one or more electrons by an atom or ion; (b) removal of oxygen from a compound; (c) addition of hydrogen to a compound. Oxidation may occur, for example, by (a) loss of one or more electrons; (b) addition of oxygen to a compound; (c) loss of hydrogen from a compound. Oxidation and reduction generally occur simultaneously (redox reactions); the substance that gains electrons is terms the oxidizing agent, while the substance that loses electrons is termed the reducing agent.

The term "redox imbalance" as used herein refers to an imbalance between reactive oxygen species and the antioxidant defense mechanisms of a cell.

The term "solid state defense" as used herein refers to the mechanism whereby a macromolecule binds a radical-generating compound, de-excites an excited state species, or quenches a free radical.

The term "syndrome," as used herein, refers to a pattern of symptoms indicative of some disease or condition.

As used herein the term "treating" includes abrogating, substantially inhibiting, slowing or reversing the progression of a disease, condition or disorder, substantially ameliorating clinical or symptoms of a disease, condition or disorder, and substantially preventing the appearance of clinical or symptoms of a disease, condition or disorder. Treating further refers to accomplishing one or more of the following: (a) reducing the severity of the disease, condition or disorder; (b) limiting development of symptoms characteristic of the disease, condition or disorder(s) being treated; (c) limiting worsening of symptoms characteristic of the disease, condition or disorder(s) being treated; (d) limiting recurrence of the disease, condition or disorder(s) in patients that have previously had the disease, condition or disorder(s); and (e) limiting recurrence of symptoms in patients that were previously asymptomatic for the disease, condition or disorder(s).

The terms "VEGF-1" or "vascular endothelial growth factor-1" are used interchangeably herein to refer to a cytokine that mediates numerous functions of endothelial cells including proliferation, migration, invasion, survival, and permeability. Although VEGF is a critical regulator in physiological angiogenesis, it also plays a significant role in skeletal growth and repair.

In one embodiment of the present disclosure the composition of the present invention comprises a therapeutically effective amount of NAC, a NAC derivative, or a pharmaceutically acceptable salt of NAC and a pharmaceutically acceptable carrier. The therapeutically effective amount is effective to preserve pulmonary function or to improve lung function by, for example, reducing inflammation, reducing or reversing symptoms, extending the time between exacerbations or a combination thereof. The term "improving or preserving lung function" in its various grammatical forms as used herein means increasing or maintaining the performance of the lung in a patient affected by a pulmonary disorder. For example, lung function can be measured by pulmonary function tests using equipment designed for this purpose. Known techniques used in the art to monitor lung function include, but are not limited to, spirometry, which provides information about airflow limitation and lung volumes; plethysmography, which provides information about airway resistance, total lung size, and trapped gas; transfer factor, which provides information about alveolar function; gas washout tests, which provide information about gas mixing, small airway function, and heterogeneous changes in compliance; computational tomography, which provides information about large and small airway deterioration; and oscillometry, which may provide information about small airways.

As used herein the terms ""therapeutically effective amount" or "pharmaceutically effective amount" refer to the amount of the compositions of the invention that result in a therapeutic or beneficial effect following its administration to a subject. The concentration of the substance is selected so as to exert its therapeutic effect, but low enough to avoid significant side effects within the scope and sound judgment of the skilled artisan. The effective amount of the composition may vary with the age and physical condition of the biological subject being treated, the severity of the condition, the duration of the treatment, the nature of concurrent therapy, the specific compound, composition or other active ingredient employed, the particular carrier utilized, and like factors. According to one embodiment, the therapeutically effective amount comprises at least 200 mg of N-acetylcysteine, the derivative of N-acetylcysteine, or the pharmaceutically acceptable salt of N-acetylcysteine but less than 20,000 mg thereof.

A skilled artisan may determine a therapeutically effective amount of the compositions described herein by determining the unit dose. As used herein, a "unit dose" refers to the amount of a composition required to produce a response of 50% of maximal effect (i.e. ED50). The unit dose may be assessed by extrapolating from dose-response curves derived from in vitro or animal model test systems. The amount of the NAC compounds in the compositions of the present invention which will be effective in the treatment of a particular disorder or condition will depend on the nature of the disorder or condition, and may be determined by standard clinical techniques. (See, for example, Goodman and Gilman's THE PHARMACOLOGICAL BASIS OF THERAPEUTICS, Joel G. Harman, Lee E. Limbird, Eds.; McGraw Hill, New York, 2001; THE PHYSICIAN'S DESK REFERENCE, Medical Economics Company, Inc., Oradell, N.J., 1995; and DRUG FACTS AND COMPARISONS, FACTS AND COMPARISONS, INC., St. Louis, Mo., 1993). The precise dose to be employed in the formulation will also depend on the route of administration, and the seriousness of the disease or disorder, and should be decided according to the judgment of the practitioner and each patient's circumstances.

The term "pharmaceutical composition," as used herein, refers to a composition that has under gone federal regulatory review, which prevents, reduces in intensity, cures, ameliorates, or otherwise treats a target disorder or disease.

According to another embodiment, the pharmaceutical composition is a tablet. According to another embodiment, N-acetylcysteine is formulated as an effervescent tablet dosage form. Effervescent tablets allow for an even distribution of N-acetylcysteine concentration and create a balanced buffered solution for easy absorption. According to some such embodiments, in order to protect each N-acetylcysteine effervescent tablet from degradation and oxidation, each dose of the N-acetylcysteine composition is vacuum-wrapped in foil packaging. According to another embodiment, each dose of the N-Acetylcysteine composition is vacuum-wrapped in paper packaging. According to another embodiment, each dose of the N-acetylcysteine composition is vacuum-wrapped in plastic packaging. According to some such embodiments, each tablet contains about 900 mg of N-acetylcysteine. According to another embodiment, each tablet contains about 600 mg of N-acetylcysteine.

According to another embodiment, the therapeutically effective amount for oral administration ranges from about 900 mg of N-acetylcysteine, the derivative of N-acetylcysteine, or the pharmaceutically acceptable salt of N-acetylcysteine per day to about 2,700 mg of N-acetylcysteine, the derivative of N-acetylcysteine or the pharmaceutically acceptable salt of N-acetylcysteine per day. Ranges, in various aspects, are expressed herein as from "about" or "approximately" one particular value and/or to "about" or "approximately" another particular value. When values are expressed as approximations by use of the antecedent "about," it will be understood that some amount of variation is included in the range.

According to another embodiment, the patient is administered orally about 900 mg of N-acetylcysteine, the derivative of N-acetylcysteine or the pharmaceutically acceptable salt of N-acetylcysteine each time, three times a day. According to another embodiment, the method further comprises monitoring at least one lung function before treatment and at intervals (e.g., 4, 8, 12, 16, 20, and 24 weeks) following treatment. According to another embodiment, the method further comprises monitoring at least one lung function before chronic treatment. According to another embodiment, the method further comprises monitoring at least one lung function at intervals (e.g., 4, 8, 12, 16, 20, and 24 weeks) following chronic treatment. According to another embodiment, the method further comprises monitoring at least one lung function during chronic treatment. According to another embodiment, the method further comprises monitoring at least one lung function before acute treatment. According to another embodiment, the method further comprises monitoring at least one lung function at intervals following acute treatment. According to another embodiment, the method further comprises monitoring at least one lung function during acute treatment.

The terms "drug carrier", "carrier", or "vehicle" as used herein refers to a pharmaceutically acceptable inert agent or vehicle for delivering one or more active agents to a mammal, and often is referred to as "excipient." As used herein the term "a pharmaceutically acceptable carrier" refers to any substantially non-toxic carrier conventionally useable for NAC administration in which NAC will remain stable and bioavailable. The carrier suitable for NAC administration must be of sufficiently high purity and of sufficiently low toxicity to render it suitable for administration to the mammal being treated. Carriers and vehicles useful herein include any such materials known in the art which are nontoxic and do not interact with other components. The (pharmaceutical) carrier may be, without limitation, a binding agent (e.g., pregelatinized maize starch, polyvinylpyrrolidone or hydroxypropyl methylcellulose, etc.), a filler (e.g., lactose and other sugars, microcrystalline cellulose, pectin, gelatin, calcium sulfate, ethyl cellulose, polyacrylates, calcium hydrogen phosphate, etc.), a lubricant (e.g., magnesium stearate, talc, silica, colloidal silicon dioxide, stearic acid, metallic stearates, hydrogenated vegetable oils, corn starch, polyethylene glycols, sodium benzoate, sodium acetate, etc.), a disintegrant (e.g., starch, sodium starch glycolate, etc.), or a wetting agent (e.g., sodium lauryl sulphate, etc.). Other suitable (pharmaceutical) carriers for the compositions of the present invention include, but are not limited to, water, salt solutions, alcohols, polyethylene glycols, gelatins, amyloses, magnesium stearates, talcs, silicic acids, viscous paraffins, hydroxymethylcelluloses, polyvinylpyrrolidones and the like.

In some embodiments, the carrier of the composition of the present invention includes a release agent such as sustained release or delayed release carrier. In such embodiments, the carrier may be any material capable of sustained or delayed release to provide a more efficient administration, e.g., resulting in less frequent and/or decreased dosage, improve ease of handling, and extend or delay effects on diseases, disorders, conditions, syndromes, and the like, being treated. Non-limiting examples of such carriers include liposomes, microsponges, microspheres, or microcapsules of natural and synthetic polymers and the like. Liposomes may be formed from a variety of phospholipids such as cholesterol, stearylamines or phosphatidylcholines.

The NAC used should be substantially free of sulfones or other chemicals that interfere with the metabolism of any co-administered drug in its bioactive form, substantially free of its oxidized form, di-N-acetylcysteine, and the composition should be prepared in a manner that substantially prevents oxidation of the NAC during preparation or storage.

It may be noted that the effectiveness of NAC depends on the presence of the reduced form, which may, for example, liberate the reduced form of glutathione from homo- and hetero-disulfide derivatives in thiol-disulfide exchange reactions. A typical unit dosage may be a solution suitable for oral or intravenous administration; an effervescent tablet suitable for dissolving in water, fruit juice, or carbonated beverage and administered orally; a tablet taken from two to six times daily, or one time-release capsule or tablet taken several times a day and containing a proportionally higher content of active ingredient, etc. The time-release effect may be obtained by capsule materials that dissolve at different pH values, by capsules that release slowly by osmotic pressure, or by any other known means of controlled release. Unit dosage forms may be provided wherein each dosage unit, for example, teaspoonful, tablespoonful, gel capsule, tablet or suppository, contains a predetermined amount of the compositions of the present invention. Similarly, unit dosage forms for injection or intravenous administration may comprise the compound of the present invention in a composition as a solution in sterile water, normal saline or another pharmaceutically acceptable carrier. The specifications for the unit dosage forms of the present invention depend on the effect to be achieved and the intended recipient. Thus, in some embodiments, NAC is formulated at high doses as an effervescent tablet or in granular form in a single dose packet to be dissolved in water to prevent untoward stomach effects.

Over-the-counter NAC may be variably produced and packaged. Because the production and packaging methods generally do not guard against oxidation, the NAC may be significantly contaminated with bioactive oxidation products. These may be particularly important in view of data indicating that the oxidized form of NAC has effects counter to those reported for NAC and is bioactive at doses roughly 10-100 fold less than NAC. See Sarnstrand et al., J. Pharmacol. Exp. Ther. 288:1174-84 (1999).

The distribution of the oxidation states of NAC as a thiol and disulfide depends on the oxidation/reduction (redox) potential. The half-cell potential obtained for the NAC thiol/disulfide pair is about +63 mV, indicative of its strong reducing activity among natural compounds [see Noszal et al. J. Med. Chem. 43:2176-2182 (2000)]. In one embodiment of the invention, the preparation and storage of the formulation is performed in such a way that the reduced form of NAC is the primary form administered to the patient. Maintaining NAC containing formulations in solid form is preferable for this purpose. When in solution, NAC containing formulations are preferably stored in a brown bottle that is vacuum sealed. Storage in cool dark environments is also preferred.

The determination of reduced and oxidized species present in a sample may be determined by various methods known in the art, including, but not limited to, for example, capillary electrophoresis, and high performance liquid chromatography as described by Chassaing et al. J. Chromatogr. B. Biomed. Sci. Appl. 735(2):219-27 (1999)..

The compositions of the present invention may be administered systemically either orally, parenterally, or rectally in dosage unit formulations containing conventional nontoxic pharmaceutically acceptable carriers, adjuvants, and vehicles as desired.

The compositions of the present invention may be in a form suitable for oral use, for example, as tablets, troches, lozenges, aqueous or oily suspensions, dispersible powders or granules, emulsions, hard or soft capsules or syrups or elixirs. Compositions intended for oral use may be prepared according to any known method, and such compositions may contain one or more agents selected from the group consisting of sweetening agents, flavoring agents, coloring agents, and preserving agents in order to provide pharmaceutically elegant and palatable preparations. Tablets may contain the active ingredient(s) in admixture with non-toxic pharmaceutically-acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch or alginic acid; binding agents, for example, starch, gelatin or acacia; and lubricating agents, for example, magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They also may be coated for controlled release.

Compositions of the present invention also may be formulated for oral use as hard gelatin capsules, where the active ingredient(s) is(are) mixed with an inert solid diluent, for example, calcium carbonate, calcium phosphate or kaolin, or soft gelatin capsules wherein the active ingredient(s) is (are) mixed with water or an oil medium, for example, peanut oil, liquid paraffin, or olive oil.

The compositions of the present invention may be formulated as aqueous suspensions wherein the active ingredient(s) is (are) in admixture with excipients suitable for the manufacture of aqueous suspensions. Such excipients are suspending agents, for example, sodium carboxymethylcellulose, methylcellulose, hydroxy-propylmethylcellulose, sodium alginate, polyvinylpyrrolidone, gum tragacanth, and gum acacia; dispersing or wetting agents may be a naturally-occurring phosphatide such as lecithin, or condensation products of an alkylene oxide with fatty acids, for example, polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions also may contain one or more coloring agents, one or more flavoring agents, and one or more sweetening agents, such as sucrose or saccharin.

Compositions of the present invention may be formulated as oily suspensions by suspending the active ingredient in a vegetable oil, for example arachis oil, olive oil, sesame oil or coconut oil, or in a mineral oil, such as liquid paraffin. The oily suspensions may contain a thickening agent, for example, beeswax, hard paraffin or cetyl alcohol. Sweetening agents, such as those set forth above, and flavoring agents may be added to provide a palatable oral preparation. These compositions may be preserved by the addition of an antioxidant such as ascorbic acid.

Compositions of the present invention may be formulated in the form of dispersible powders and granules suitable for preparation of an aqueous suspension by the addition of water. The active ingredient in such powders and granules is provided in admixture with a dispersing or wetting agent, suspending agent, and one or more preservatives. Suitable dispersing or wetting agents and suspending agents are exemplified by those already mentioned above. Additional excipients, for example, sweetening, flavoring and coloring agents also may be present.

Compositions of the invention also may be formulated as a beverage or as an additive to a beverage, where the term "beverage" refers to any non-alcoholic flavored carbonated drink, soda water, non-alcoholic still drinks, diluted fruit or vegetable juices whether sweetened or unsweetened, seasoned or unseasoned with salt or spice, or still or carbonated mineral waters used as a drink. The term "additive" as used herein refers to any substance the intended use of which results, or may reasonably be expected to result, directly or indirectly, in its becoming a component or otherwise affecting the characteristics of any beverage. In some embodiments, the beverage is a flavored carbonated beverage. In some embodiments, the beverage is a flavored non-carbonated beverage. In some embodiments, the beverage is a natural fruit beverage. The beverage also may contain one or more coloring agents, one or more flavoring agents, one or more sweetening agents, one or more antioxidant agents, and one or more preservatives.

Compositions of the invention also may be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example, gum acacia or gum tragacanth, naturally-occurring phosphatides, for example soy bean, lecithin, and esters or partial esters derived from fatty acids and hexitol anhydrides, for example sorbitan monooleate, and condensation products of the partial esters with ethylene oxide, for example, polyoxyethylene sorbitan monooleate. The emulsions also may contain sweetening and flavoring agents.

Compositions of the invention also may be formulated as syrups and elixirs. Syrups and elixirs may be formulated with sweetening agents, for example, glycerol, propylene glycol, sorbitol or sucrose. Such formulations also may contain a demulcent, a preservative, and flavoring and coloring agents. Demulcents are protective agents employed primarily to alleviate irritation, particularly mucous membranes or abraded tissues. A number of chemical substances possess demulcent properties. These substances include the alginates, mucilages, gums, dextrins, starches, certain sugars, and polymeric polyhydric glycols. Others include acacia, agar, benzoin, carbomer, gelatin, glycerin, hydroxyethyl cellulose, hydroxypropyl cellulose, hydroxypropyl methylcellulose, propylene glycol, sodium alginate, tragacanth, hydrogels and the like.

For buccal administration, the compositions of the present invention may take the form of tablets or lozenges formulated in a conventional manner.

The compositions of the present invention may be in the form of a sterile injectable aqueous or oleaginous suspension. The term "parenteral" as used herein includes subcutaneous injections, intravenous, intramuscular, intrasternal injection, or infusion techniques. Injectable preparations, such as sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1, 3-butanediol. Among the acceptable vehicles and solvents that may be employed are water, Ringer's solution, and isotonic sodium chloride solution. In addition, sterile, fixed oils are conventionally employed as a solvent or suspending medium. For parenteral application, particularly suitable vehicles consist of solutions, preferably oily or aqueous solutions, as well as suspensions, emulsions, or implants. Aqueous suspensions may contain substances which increase the viscosity of the suspension and include, for example, sodium carboxymethyl cellulose, sorbitol and/or dextran. Optionally, the suspension may also contain stabilizers.

The term "topical" refers to administration of an inventive composition at, or immediately beneath, the point of application. The phrase "topically applying" describes application onto one or more surfaces(s) including epithelial surfaces. Topical administration generally provides a local rather than a systemic effect. For the purpose of this application, topical applications shall include mouthwashes and gargles.

Topical administration may also involve the use of transdermal administration such as transdermal patches or iontophoresis devices which are prepared according to techniques and procedures well known in the art. The terms "transdermal delivery system", transdermal patch" or "patch" refer to an adhesive system placed on the skin to deliver a time released dose of a drug(s) by passage from the dosage form through the skin to be available for distribution via the systemic circulation. Transdermal patches are a well-accepted technology used to deliver a wide variety of pharmaceuticals, including, but not limited to, scopolamine for motion sickness, nitroglycerin for treatment of angina pectoris, clonidine for hypertension, estradiol for postmenopausal indications, and nicotine for smoking cessation.

Patches suitable for use in the present invention include, but are not limited to, (1) the matrix patch; (2) the reservoir patch; (3) the multi-laminate drug-in-adhesive patch; and (4) the monolithic drug-in-adhesive patch; TRANSDERMAL AND TOPICAL DRUG DELIVERY SYSTEMS, pp. 249-297 (Tapash K. Ghosh et al. eds., 1997). These patches are well known in the art and generally available commercially.

The compositions of the present invention may be in the form of a dispersible dry powder for pulmonary delivery. Dry powder compositions may be prepared by processes known in the art, such as lyophilization and jet milling, as disclosed in International Patent Publication No. WO 91/16038 and as disclosed in U.S. Pat. No. 6,921,527. The composition of the present invention is placed within a suitable dosage receptacle in an amount sufficient to provide a subject with a unit dosage treatment. The dosage receptacle is one that fits within a suitable inhalation device to allow for the aerosolization of the dry powder composition by dispersion into a gas stream to form an aerosol and then capturing the aerosol so produced in a chamber having a mouthpiece attached for subsequent inhalation by a subject in need of treatment. Such a dosage receptacle includes any container enclosing the composition known in the art such as gelatin or plastic capsules with a removable portion that allows a stream of gas (e.g., air) to be directed into the container to disperse the dry powder composition. Such containers are exemplified by those shown in U.S. Pat. Nos. 4,227,522; U.S. Pat. No. 4,192,309; and U.S. Pat. No. 4,105,027. Suitable containers also include those used in conjunction with Glaxo's Ventolin® Rotohaler brand powder inhaler or Fison's Spinhaler® brand powder inhaler. Another suitable unit-dose container which provides a superior moisture barrier is formed from an aluminum foil plastic laminate. The pharmaceutical-based powder is filled by weight or by volume into the depression in the formable foil and hermetically sealed with a covering foil-plastic laminate. Such a container for use with a powder inhalation device is described in U.S. Pat. No. 4,778,054 and is used with Glaxo's Diskhaler® (U.S. Pat. Nos. 4,627,432; 4,811,731; and 5,035,237).

The compositions of the present invention may be in the form of suppositories for rectal administration of the composition. These compositions may be prepared by mixing the drug with a suitable nonirritating excipient such as cocoa butter and polyethylene glycols which are solid at ordinary temperatures but liquid at the rectal temperature and will therefore melt in the rectum and release the drug. When formulated as a suppository the compositions of the invention may be formulated with traditional binders and carriers, such as triglycerides.

The therapeutically active agent of the present invention may be formulated per se or in salt form. The term "pharmaceutically acceptable salt" as used herein refers to those salts which are, within the scope of sound medical judgment, suitable for use in contact with the tissues of humans and lower animals without undue toxicity, irritation, allergic response and the like and are commensurate with a reasonable benefit/risk ratio. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulfuric, nitric, phosphoric, maleic, acetic, salicylic, p-toluene sulfonic, tartaric, citric, methane sulfonic, formic, malonic, succinic, naphthalene-2-sulfonic, and benzene sulfonic. Also, such salts may be prepared as alkaline metal or alkaline earth salts, such as sodium, potassium or calcium salts of the carboxylic acid group. By "pharmaceutically acceptable salt" is meant those salts which are, within the scope of sound medical judgment, suitable for use in contact with the tissues of humans and lower animals without undue toxicity, irritation, allergic response and the like and are commensurate with a reasonable benefit/risk ratio. Pharmaceutically acceptable salts are well-known in the art. For example, P. H. Stahl, et al. describe pharmaceutically acceptable salts in detail in "Handbook of Pharmaceutical Salts: Properties, Selection, and Use" (Wiley VCH, Zurich, Switzerland: 2002). The salts may be prepared in situ during the final isolation and purification of the compounds described within the present invention or separately by reacting a free base function with a suitable organic acid. Representative acid addition salts include, but are not limited to, acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate(isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups may be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid. Basic addition salts may be prepared in situ during the final isolation and purification of compounds described within the invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like.
Pharmaceutically acceptable salts also may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium or magnesium) salts of carboxylic acids may also be made. Additional compositions of the present invention may be readily prepared using technology which is known in the art such as described in Remington's Pharmaceutical Sciences, 18th or 19th editions, published by the Mack Publishing Company of Easton, Pennsylvania.

The present invention further provides a pharmaceutical pack or kit comprising one or more containers filled with one or more of the ingredients of the pharmaceutical compositions of the invention. Associated with such container(s) may be a notice in the form prescribed by a governmental agency regulating the manufacture, use or sale of pharmaceuticals or biological products, which notice reflects approval by the agency of manufacture, use or sale for human administration.

For example, in one embodiment, a pharmaceutical kit for treating inflammation in cystic fibrosis patients according to the present invention includes a first container filled with a pharmaceutically effective amount of a cystic fibrosis therapeutic agent and a second container filled with a composition comprising a therapeutically effective amount of N-acetylcysteine or a pharmaceutically acceptable salt of N-acetylcysteine, and a pharmaceutically acceptable carrier.

In another embodiment, a pharmaceutical kit for preserving or restoring lung function in cystic fibrosis patients according to the present invention includes a first container filled with a pharmaceutically effective amount of a cystic fibrosis therapeutic agent and a second container filled with a composition comprising a therapeutically effective amount of N-acetylcysteine or a pharmaceutically acceptable salt of N-acetylcysteine, and a pharmaceutically acceptable carrier.

In yet another embodiment, a pharmaceutical kit for prolonging the time interval between exacerbations in cystic fibrosis patients according to the present invention includes a first container filled with a pharmaceutically effective amount of a cystic fibrosis therapeutic agent and a second container filled with a composition comprising a therapeutically effective amount of N-acetylcysteine or a pharmaceutically acceptable salt of N-acetylcysteine, and a pharmaceutically acceptable carrier.

Regular, routine treatment to keep secretions cleared and prevent infection is very important in CF because respiratory complications are the leading cause of morbidity and mortality in CF patients. Thick mucus blocks the bronchial tubes in the lungs leading to inflammation and recurrent infections. With each infection, more damage or scarring occurs, causing lung function to progressively worsen.

According to one embodiment, the composition of the present invention reverses at least one symptom of the pulmonary disorder that includes an inflammatory component. Symptoms associated with, for example, cystic fibrosis, include but are not limited to chronic coughing, difficulty breathing, pneumonia caused by abnormal mucus in respiratory and gastrointestinal tracts, lung obstruction, infection, poor digestion, poor food absorption, etc.

In some embodiments known techniques are used to monitor lung function. Such known techniques include, but are not limited to spirometry, plethysmography, transfer factor, gas washout tests, computational tomography, and oscillometry. Exemplary endpoints that reflect lung function include, but are not limited to, FEV1, FEF, FVC, etc.

In some embodiments, administration of a pharmaceutical composition comprising a therapeutically effective amount of N-acetylcysteine or a pharmaceutically acceptable salt of N-acetylcysteine, increases Forced Expiratory Volume in one second (FEV₁) value of a treated patient from about at least 0.1 % to about at least 99%, as compared to the FEV₁ value of an untreated control patient. In one embodiment, administration of the pharmaceutical composition increases the FEV₁ value of a treated patient at least 0.1% as compared to the FEV₁ value of an untreated control patient. In one embodiment, administration of the pharmaceutical composition increases the FEV₁ value of a treated patient at least 0.5% as compared to the FEV₁ value of an untreated control patient. In one embodiment, administration of the pharmaceutical composition increases the FEV₁ value of a treated patient at least 1.0 % as compared to the FEV₁ value of an untreated control patient. In
one embodiment, administration of the pharmaceutical composition increases the FEV₁ value of a treated patient at least 2.0 % as compared to the FEV₁ value of an untreated control patient. In one embodiment, administration of the pharmaceutical composition increases the FEV₁ value of a treated patient at least 3.0 % as compared to the FEV₁ value of an untreated control patient. In one embodiment, administration of the pharmaceutical composition increases the FEV₁ value of a treated patient at least 4.0 % as compared to the FEV₁ value of an untreated control patient. In one embodiment, administration of the pharmaceutical composition increases the FEV₁ value of a treated patient at least 5.0 % as compared to the FEV₁ value of an untreated control patient. In one embodiment, administration of the pharmaceutical composition increases the FEV₁ value of a treated patient at least 10.0 % as compared to the FEV₁ value of an untreated control patient. In one embodiment, administration of the pharmaceutical composition increases the FEV₁ value of a treated patient at least 15.0 % as compared to the FEV₁ value of an untreated control patient. In one embodiment, administration of the pharmaceutical composition increases the FEV₁ value of a treated patient at least 20.0 % as compared to the FEV₁ of an untreated control patient. In one embodiment, administration of the pharmaceutical composition increases the FEV₁ of a treated patient at least 25.0 % as compared to the FEV₁ value of an untreated control patient. In one embodiment, administration of the pharmaceutical composition increases the FEV₁ value of a treated patient at least 30.0 % as compared to the FEV₁ value of an untreated control patient. In one embodiment, administration of the pharmaceutical composition increases the FEV₁ value of a treated patient at least 35.0 % as compared to the FEV₁ value of an untreated control patient. In one embodiment, administration of the pharmaceutical composition increases the FEV₁ value of a treated patient at least 40.0 % as compared to the FEV₁ value of an untreated control patient. In one embodiment, administration of the pharmaceutical composition increases the FEV₁ value of a treated patient at least 45.0 % as compared to the FEV₁ value of an untreated control patient. In one embodiment, administration of the pharmaceutical composition increases the FEV₁ value of a treated patient at least 50.0 % as compared to the FEV₁ value of an untreated control patient. In one embodiment, administration of the pharmaceutical composition increases the FEV₁ value of a treated patient at least 60.0 % as compared to the FEV₁ of an untreated control patient. In one embodiment, administration of the pharmaceutical composition increases the FEV₁ of a treated patient at least 70.0 % as compared to the FEV₁ of an untreated control patient. In one embodiment, administration of the pharmaceutical composition increases the FEV₁ value of a treated patient at least 80.0 % as compared to the FEV₁ value of an untreated control patient. In one embodiment, administration of the pharmaceutical composition increases the FEV₁ value of a treated patient at least 90.0 % as compared to the FEV₁ value of an untreated control patient. In one embodiment, administration of the pharmaceutical composition increases the FEV₁ value of a treated patient at least 99.0 % as compared to the FEV₁ value of an untreated control patient.

In some embodiments, administration of a pharmaceutical composition comprising a therapeutically effective amount of N-acetylcysteine or a pharmaceutically acceptable salt of N-acetylcysteine, increases Forced Expiratory Volume in one second (FEV₁) value measured in a patient following treatment from about at least 0.1 % to about at least 99%, as compared to a baseline FEV₁ value measured in the patient measured before treatment. In one embodiment, administration of the pharmaceutical composition increases the FEV₁ value measured in a patient following treatment by at least 0.1% as compared to a baseline FEV₁ value measured in the patient measured before treatment. In one embodiment, administration of the pharmaceutical composition increases the FEV₁ value measured in a patient following treatment by at least 0.5% as compared to a baseline FEV₁ value measured in the patient measured before treatment. In one embodiment, administration of the pharmaceutical composition increases the FEV₁ value measured in a patient following treatment by at least 1.0 % as compared to a baseline FEV₁ value measured in the patient measured before treatment. In one embodiment, administration of the pharmaceutical composition increases the FEV₁ value measured in a patient following treatment by at least 2.0 % as compared to a baseline FEV₁ value measured in the patient measured before treatment. In one embodiment, administration of the pharmaceutical composition increases the FEV₁ value measured in a patient following treatment by at least 3.0 % as compared to a baseline FEV₁ value measured in the patient measured before treatment. In one embodiment, administration of the pharmaceutical composition increases the FEV₁ value measured in a patient following treatment by at least 4.0 % as compared to a baseline FEV₁ value measured in the patient measured before treatment. In one embodiment, administration of the pharmaceutical composition increases the FEV₁ value measured in a patient following treatment by at least 5.0 % as compared to a baseline FEV₁ value measured in the patient measured before treatment. In one embodiment, administration of the pharmaceutical composition increases the FEV₁ value measured in a patient following treatment by at least 10.0 % as compared to a baseline FEV₁ value measured in the patient measured before
treatment. In one embodiment, administration of the pharmaceutical composition increases the FEV₁ value measured in a patient following treatment by at least 15.0 % as compared to a baseline FEV₁ value measured in the patient measured before treatment. In one embodiment, administration of the pharmaceutical composition increases the FEV₁ value measured in a patient following treatment by at least 20.0 % as compared to a baseline FEV₁ value measured in the patient measured before treatment. In one embodiment, administration of the pharmaceutical composition increases the FEV₁ value measured in a patient following treatment by at least 25.0 % as compared to a baseline FEV₁ value measured in the patient measured before treatment. In one embodiment, administration of the pharmaceutical composition increases the FEV₁ value measured in a patient following treatment by at least 30.0 % as compared to a baseline FEV₁ value measured in the patient measured before treatment. In one embodiment, administration of the pharmaceutical composition increases the FEV₁ value measured in a patient following treatment by at least 35.0 % as compared to a baseline FEV₁ value measured in the patient measured before treatment. In one embodiment, administration of the pharmaceutical composition increases the FEV₁ value measured in a patient following treatment by at least 40.0 % as compared to a baseline FEV₁ value measured in the patient measured before treatment. In one embodiment, administration of the pharmaceutical composition increases the FEV₁ value measured in a patient following treatment by at least 45.0 % as compared to a baseline FEV₁ value measured in the patient measured before treatment. In one embodiment, administration of the pharmaceutical composition increases the FEV₁ value measured in a patient following treatment by at least 50.0 % as compared to a baseline FEV₁ value measured in the patient measured before treatment. In one embodiment, administration of the pharmaceutical composition increases the FEV₁ value measured in a patient following treatment by at least 60.0 % as compared to a baseline FEV₁ value measured in the patient measured before treatment. In one embodiment, administration of the pharmaceutical composition increases the FEV₁ value measured in a patient following treatment by at least 70.0 % as compared to a baseline FEV₁ value measured in the patient measured before treatment. In one embodiment, administration of the pharmaceutical composition increases the FEV₁ value measured in a patient following treatment by at least 80.0 % as compared to a baseline FEV₁ value measured in the patient measured before treatment. In one embodiment, administration of the pharmaceutical composition increases the FEV₁ value measured in a patient following treatment by at least 90.0 % as compared to a baseline FEV₁ value measured in the patient measured before treatment. In one embodiment, administration of the pharmaceutical composition increases the FEV₁ value measured in a patient following treatment by at least 99.0 % as compared to a baseline FEV₁ value measured in the patient measured before treatment.

In another embodiment of the present invention, compositions and methods of the present invention may be used in combination with known therapeutic agents, provided that they are compatible with each other. "Compatible" as used herein means that the compositions and methods of the present invention are capable of being combined with existing therapies in a manner such that there is no interaction that would substantially reduce the efficacy of either the compositions or methods of the present invention or the therapies under ordinary use conditions.

In some embodiments, existing cystic fibrosis therapeutic agents that may be combined with the compositions and methods of the present invention include, but are not limited to, anti-infective agents, bronchodilating agents, and anti-inflammatory agents.

Lung and airway infections in cystic fibrosis may be treated with potent anti-infective agents, including antibiotics, to improve lung function, reduce days spent in the hospital and to reduce use of intravenous antibiotics to reduce bacterial levels in the lungs. Inhaled antibiotics also are used to prevent lung infections that may lead to hospitalization.

To minimize certain side effects, bronchodilating agents often are used along with inhaled antibiotics. Bronchodilating agents are used widely for treating a variety of obstructive lung diseases, including cystic fibrosis. They relax smooth muscle in the small airways of the lungs, which dilates the airways and makes breathing easier, particularly when airways are narrowed by inflammation. Inhaled bronchodilator medications used in asthma, such as albuterol, have improved breathing in some people with cystic fibrosis. When used to treat cystic fibrosis, bronchodilating agents are usually given through a nebulizer or with a handheld inhaler. Airway dilatation before physiotherapy helps the cystic fibrosis patient to clear chest secretions.

Nonsteroidal anti-inflammatory agents reduce inflammation and pain. Cystic fibrosis patients often have persistent lung inflammation which becomes part of the cycle of continued lung damage in these patients. Anti-inflammatory medications, such as ibuprofen, in some patients with CF help to reduce this inflammation. In some children, anti-inflammatory medications may significantly slow the progression of lung disease and improve breathing.

In some embodiments, therapeutic agents, such as corticosteroids, anticoagulation agents, and pirfenidone, may be administered to treat the inflammation present in some patients with IPF in combination with the compositions and methods of the present invention. Antimicrobial agents also may be used to treat bacterial organisms, opportunistic pathogens, and common respiratory viruses.

In some embodiments, standard doses of existing therapeutic agents for chronic and acute exacerbations of asthma may be combined with the compositions and methods of the present invention. These include, but are not limited to, antimicrobial agents, bronchodilators (e.g., epinephrine, terbutaline, ipratropium (Atrovent®)), inhaled corticosteroids, leukotriene antagonists, β-agonists (e.g., albuterol [e.g., Ventolin ®, Proventil®], levalbuterol, Metaproterenol Sulfate (Alupent), isoprotenerol, chromolyn sodium; aminophylline, and theophylline.

In another embodiment of the present invention, compositions and methods of the present invention may be used in combination with known therapies, provided that they are compatible with each other.

The term "respiratory therapy" as used herein refers to chest physiotherapy, which is used to help clear excess mucus out of the lungs. To perform chest physiotherapy, a patient is placed in various positions allowing major segments of the lungs to point downward and then clapping firmly over chest and back on part of the lung segment to shake the mucus loose. Once loosened, the mucus will fall to the large airways, where it may be coughed out. Chest physiotherapy may be time-consuming since 3-5 minutes is spent clapping over 10-12 lung segments. It is also difficult for patients to perform on themselves and usually requires a skilled caregiver.

The term "rehabilitative therapy" refers to a therapy designed to help patients use their energy more efficiently, i.e., in a way that requires less oxygen. Rehabilitative therapy improves shortness of breath and overall survival, especially in those with advanced disease.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any method and materials similar or equivalent to those described herein can also be used in the practice or testing of the present invention, the preferred methods and materials are now described. All publications mentioned herein disclose and describe the methods and/or materials in connection with which the publications are cited.

It must be noted that as used herein and in the appended claims, the singular forms "a", "and", and "the" include plural references unless the context clearly dictates otherwise. All technical and scientific terms used herein have the same meaning.

### Examples

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1. Randomized, Placebo-Controlled, Double-Blind Study of the Effects of Oral N-Acetylcysteine on Redox Changes and Lung Inflammation in CF Patients

A 24-week (6-month) multi-center large clinical trial was designed with randomization stratified to improve balance of baseline characteristics between treatment groups, and to improve precision between group treatment effects.

A 6-month multi-center phase II clinical trial was performed to evaluate the effect of N-acetylcysteine (PharmaNAC®, BioAdvantex Pharma, Mississauga, Ontario Canada)) 900 mg effervescent tablets taken orally 3 times a day on airway inflammation in CF patients. 70 subjects were randomized to placebo or study drug. Randomization was stratified by study site, baseline FEV₁, age, gender, chronic azithromycin, inhaled aztreonam for inhalation solution (AZLI) or tobramycin inhalation solution, USP (TOBI®, Novartis) and ibuprofen use. A cohort of 16 subjects served as an initial safety cohort.

General objectives of the study included determination of safety, tolerability and efficacy of NAC versus placebo at 0 week, 6 weeks, 12 week and 24 week time points. More specific objectives included evaluation of: (a) effect of NAC on number of pulmonary and sinus exacerbations and antibiotic use, (b) effect of NAC on lung inflammation; (c) effect of NAC on pulmonary function; (d) effect of NAC on weight; (e) effect of NAC on quality of life (QOL) and (f) whether NAC causes pulmonary hypertension if used chronically in high doses.

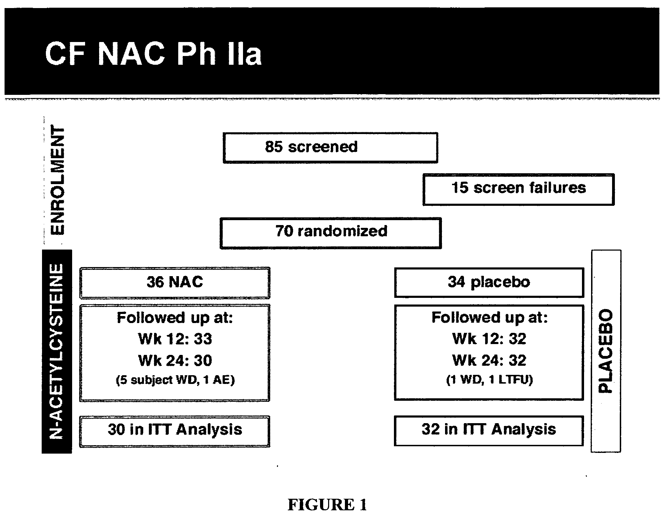

Figure 1 shows the enrollment scheme. Of 85 subjects screened for eligibility, 70 were enrolled and randomized into NAC and placebo groups of 36 and 34 subjects respectively. Of the NAC group, 33 subjects followed up at week 12, 30 subjects followed up at week 24, 1 subject withdrew from the study and 1 subject showed adverse event and hence was removed from the study. Of the placebo group, 32 subjects followed up at week 12, 32 subjects followed up at week 24, one subject withdrew from the study and one subject was removed for adverse event.

The inclusion criteria to include candidate subjects in the study included the following: (a) 7 years of age or older who are able to tolerate sputum induction and perform reproducible spirometry; (b) stable mild to moderate lung disease; (c) no use of acute antibiotics in the previous 4 weeks; and (d) no use of anti-oxidants in any form at least 6 weeks prior to and for the duration of the study, although usual doses of vitamin E and vitamin C were allowed. The exclusion criteria to exclude candidate subjects from the study included: (a) use of a nonsteroidal anti-inflammatory drug in the previous one week to assure sputum neutrophil count and elastase at baseline; (b) initiation of chronic dosing with azithromycin, ibuprofen, TOBI®, inhaled ("IH") Azhetreonam or Colistin within previous six weeks; (c) liver disease; (d) active allergic bronchopulmonary aspergillosis ("ABPA") in the previous six months; and (e) acetaminophen use in the previous three days.

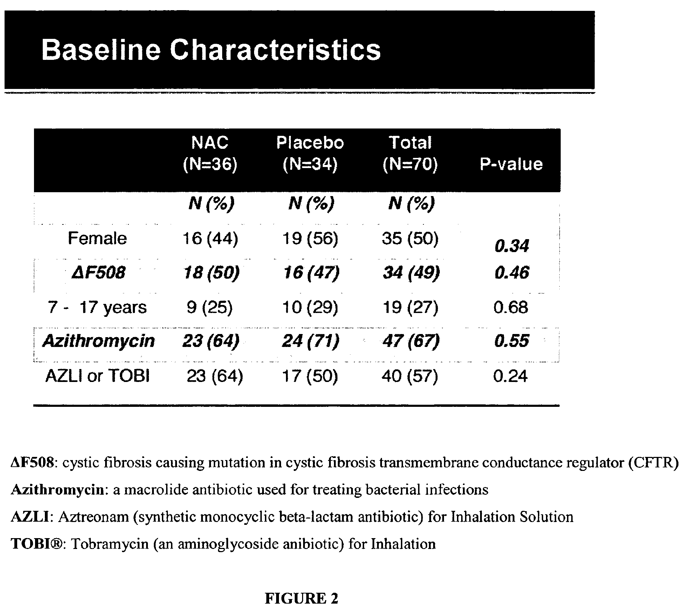

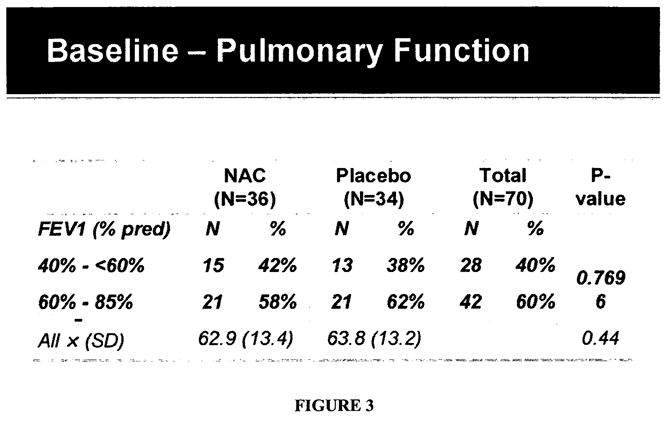

Figure 2 shows the baseline characteristics of subjects enrolled in NAC-treated and placebo-treated groups. Figure 3 shows baseline characteristics of pulmonary function in NAC-treated and placebo-treated groups. No adverse events and no pulmonary hypertension were shown in the first 16 subject safety cohort study.

The primary efficacy endpoint in this study were sputum elastase activity as measured by ELISA assay reflecting lung inflammation. Secondary efficacy endpoint were: (i) FEV₁ (liters and % Pred), reflecting lung function; (ii) sputum Human Neurophil Elastase (HNE) activity, reflecting lung inflammation, the current best predictor of CF lung disease; (iii) sputum IL-8 levels reflecting lung inflammation; (iv) whole blood GSH, reflecting redox imbalance; (v) effect of new use of antibiotics; (vi) Cystic Fibrosis Questionnaire Revised (CFQ-R) respiratory domain scores; and (vii) Cystic Fibrosis Respiratory Symptom Diary (CFRSD) scores.

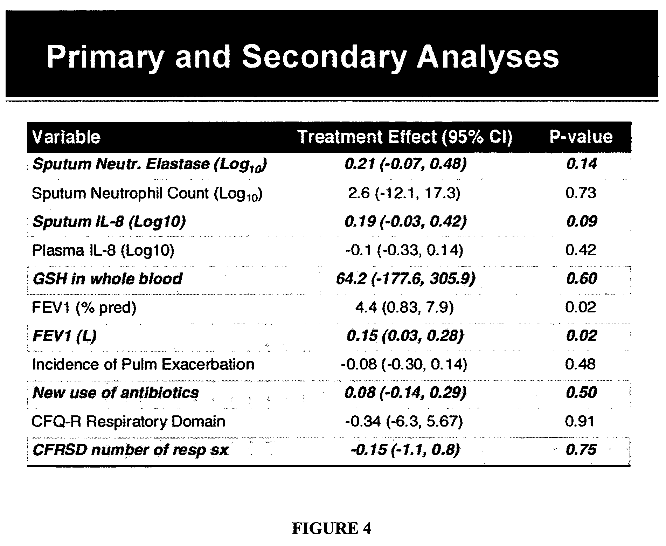

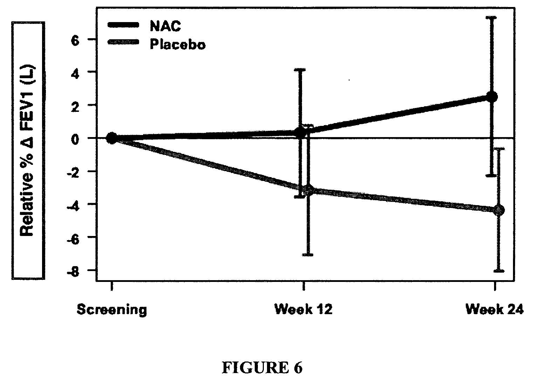

Figure 4 shows primary and secondary outcomes of NAC treatment over placebo-treated group. The NAC recipients maintained baseline FEV₁, while the placebo cohort displayed a 4% decrease in FEV₁ (150 ml) over the 6-month trial period. (P=0.02) (Figure 6).

### Example 2: Effect of NAC on chronic airway inflammation

The effect of NAC on chronic airway inflammation was measured by examining the change in the log₁₀ of neutrophil elastase activity measured in sputum from enrollment to the end of the trial. The effect of NAC on neutrophil recruitment, lung function, pulmonary and sinus exacerbations, and on weight of CF subjects was measured by: change in the sputum neutrophil count, change in IL-8 in sputum and plasma, change in GSH in whole blood, incidence of pulmonary exacerbations, number of and time to first pulmonary exacerbation, incidence and number of antibiotics for any reason, and change in FEV₁. Patient-reported outcomes (PRO) were measured by the CFQ-R and CFRD instruments.

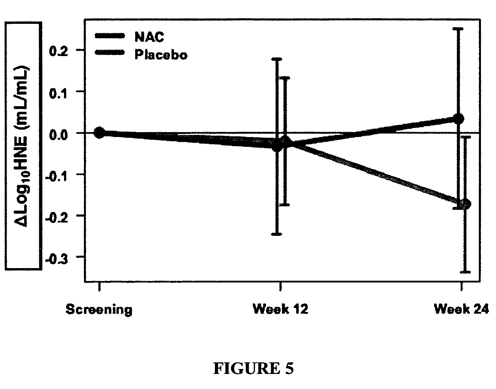

No difference was detected in airways inflammation, measured by HNE (Figure 5), number of pulmonary or sinus exacerbations, or new antibiotic prescriptions. But, NAC recipients maintained lung function over the 6-month period, while placebo recipients experienced significant loss of function as determined by FEV₁, FVC, and FEF. Without being limited by theory, NAC may exert anti-inflammatory and/or anti-oxidant effects via action in alternate pathways important in chronic inflammation, such as RANTES (Regulated upon Activation, Normal T-cell Expressed, and Secreted), IL-1β / IL-1RA, IL-17, and TGF-β.

### Example 3: Effect of long-term treatment with NAC

Effect of long-term treatment with NAC in promoting the development of pulmonary hypertension (PH) in subjects with cystic fibrosis is examined. A panel of clinical and molecular studies, performed at 6-week intervals, is assessed. This panel included level of NAC in blood, bFGF and VEGF in plasma and urine, and in the initial safety cohort, levels of S-nitrosylated NAC, HIF-1α, and ECHO and diffusing capacity of the lung for carbon monoxide (DLCO).

### Comparative Example 4. Use of NAC to treat Acute Exacerbations of IPF

A patient showing the symptoms of an acute exacerbation of IPF (including, but not limited to, idiopathic acute respiratory deterioration) may be treated with a composition comprising an acute exacerbation-reducing amount of either the purified L-enantiomer or the racemate mixture composed of equal proportions of the D- and L-isomers of NAC administered either serially or co-administered two, three or four times a day up to the highest tolerable dose, given that there will be individual variability in the ability to tolerate NAC. This dosage of NAC is sufficient to decrease key aspects of an acute exacerbation of IPF in such patients.

### Comparative Example 5. Use of NAC to treat Acute Exacerbations of Asthma

A child or adult showing the symptoms of an acute exacerbation of asthma (including, but not limited to, a sudden increase in breathlessness over the preceding 48 hours and presence of one of the following signs: tachypnea (respiratory rate of >18), use of accessory muscles or respiration, audible wheezing, prolonged expiration with rhonchi on auscultation or a silent chest) may be treated with a composition comprising at least one standard asthma therapeutic agent and an acute exacerbation-reducing amount of either the purified L-enantiomer or the racemate mixture composed of equal proportions of the D- and L-isomers of NAC administered either serially or co-administered two, three or four times a day up to the highest tolerable dose, given that there will be individual variability in the ability to tolerate NAC. This dosage of NAC is sufficient to decrease key aspects of an acute exacerbation of asthma in such patients.

### Comparative Example 6: Use of NAC to treat acute exacerbations of TB in HIV patients

An HIV patient having latent or active TB who is being treated with a formulation comprising a therapeutically effective amount of a multi-drug regimen as normally used to treat HIV and/or TB may be further treated with a composition comprising an acute exacerbation reducing amount of either the purified L-enantiomer or the racemate mixture composed of equal proportions of the D- and L-isomers of NAC administered either serially or co-administered two, three or four times a day up to the highest tolerable dose, given that there will be individual variability in the ability to tolerate NAC. This dosage of NAC is sufficient to decrease key aspects of IRIS in such patients.

### Equivalents

While the described invention has been described with reference to the specific embodiments thereof, it should be understood by those skilled in the art that various changes may be made and equivalents may be substituted without departing from the scope of the invention. In addition, many modifications may be made to adapt a particular situation, material, composition of matter, process, process step or steps, to the objective scope of the described invention.

## Claims

1. A pharmaceutical composition for use in the improvement of lung function in a patient suffering from cystic fibrosis with an inflammatory component, who is not being administered with inhaled antibiotics, said composition comprising (a) a therapeutically effective amount of N-acetylcysteine or a pharmaceutically acceptable salt thereof and (b) a carrier.

## Patentansprüche

1. Pharmazeutische Zusammensetzung zur Verwendung bei der Verbesserung der Lungenfunktion in einem Patienten, der an zystischer Fibrose mit Entzündungskomponente leidet und dem keine inhalierten Antibiotika verabreicht werden, wobei die Zusammensetzung Folgendes umfasst: (a) eine therapeutisch wirksame Menge N-Acetylcystein oder ein pharmazeutisch geeignetes Salz davon und (b) einen Träger.

## Revendications

1. Une composition pharmaceutique pour être utilisée pour l'amélioration de la fonction pulmonaire d'un patient souffrant de mucoviscidose présentant une composante inflammatoire, qui ne reçoit pas des antibiotiques administrés par inhalation, ladite composition comprenant
(a) une quantité, efficace du point de vue thérapeutique, de N-acétylcystéine ou d'un sel acceptable du point de vue pharmaceutique en dérivant, et
(b) un support.
